# EUROPEAN PATENT APPLICATION

(11) **EP 4 609 859 A1**
(43) Date of publication of application: **03.09.2025**
(21) Application number: 24382214.5
(22) Date of filing: 28.02.2024
(51) Int. Cl.: A61K 31/135, A61K 31/138, A61K 31/343, A61K 31/4422, A61K 31/445, A61K 31/4453, A61K 31/451, A61K 31/4525, A61K 31/454, A61K 31/5415, A61K 31/55, A61K 31/675, A61K 45/06, A61P 21/00

(54) **INHIBITORS OF THE GENE SMPD1 FOR THE TREATMENT OF SPINAL MUSCULAR ATROPHY**

(71) Applicant: Universidad Pablo de Olavide, 41013 Sevilla (ES); Universitat de Lleida, 25003 Lérida (ES)
(72) Inventor: PÉREZ PULIDO, Antonio J., 41013 Sevilla (ES); MUÑOZ RUIZ, Manuel Jesús, 41013 Sevilla (ES); BROKATE-LLANOS, Ana María, 41013 Sevilla (ES); GARZÓN VILLAR, Andrés, 41013 Sevilla (ES); SOLER TATCHÉ, Rosa M., 25003 Lleida (ES); GARCERÁ TERUEL, Ana, 25003 Lleida (ES)
(74) Representative: Hoffmann Eitle

(57) **Abstract**

The present invention refers to a composition comprising an inhibitory molecule capable of inhibiting the expression of a gene selected from any of the list consisting of SMPD1, ARAP1, CST3, GSN, HDAC5, PACS1, TOM1L2, TSC22D3 and USP20 or homologues thereof and increasing the levels of endogenous SMN1 and/or SMN2 proteins, or a mixture of two or more of said molecules, for use in the treatment of Spinal Muscular Atrophy (SMA) in a subject in need thereof.

## Description

### TECHNICAL FIELD

The present invention is directed to the medical field, in particular to a composition comprising an inhibitory molecule capable of inhibiting the expression of a gene selected from any of the list consisting of SMPD1, ARAP1, CST3, GSN, HDAC5, PACS1, TOM1L2, TSC22D3 and USP20 or homologues thereof and increasing the levels of endogenous SMN1 and/or SMN2 proteins, or a mixture of two or more of said molecules, for use in the treatment of Spinal Muscular Atrophy (SMA) in a subject in need thereof.

### BACKGROUND ART

Spinal Muscular Atrophy (SMA) is a rare genetic disease affecting 1 in 8,000 births, having a carrier frequency of 1 in 40 individuals (Wirth et al., 2006) and the highest rate of neonatal death among all human hereditary diseases (Lunn and Wang, 2008). It is a progressive neuromuscular disease characterized physiologically by motor neuron (MN) degeneration and skeletal muscle atrophy. At the genetic level, it is primarily caused by recessive deletion or mutation of the Survival Motor Neuron 1 (SMN1) gene (GeneID: 6606), leading to a deficiency of the survival motor neuron (SMN) protein, which is primarily involved in the assembly of small nuclear ribonucleoproteins (snRNPs) (Fischer et al., 1997). Although the latter function is ubiquitous in the organism and essential for the splicing of many messenger RNAs (mRNAs), the phenotype of the mutation is only observed specifically in motor neurons and muscles in a mainly secondary manner. In the past years, biological processes in which SMN is also involved have been discovered and are being characterized, including mRNA transport through neuronal axons, neurotransmitter vesicle trafficking, or endoplasmic reticulum and vesicular network stress pathways of the Golgi apparatus, all of which have a strong involvement of local mRNA metabolism (Fallini et al., 2012; Khalil et al., 2018). This has led to current knowledge of proteins that interact with or are part of the SMN complex, and that can modify the SMA phenotype, such as PLS3, NCALD, UBA1 or ELAVL4, none of which has so far led to the development of an efficient drug against SMA in humans (Hosseinibarkooie et al., 2017; McCabe, 2017).

The SMN1 gene was linked to SMA in 1995 (Lefebvre et al., 1995), and later a second SMN gene (GeneID: 6607) was discovered and named SMN2 (Chen et al., 1998). This latter differs in only 5 nucleotides from SMN1, one of which causes most of the SMN2 mRNA (90%) to be missing exon 7, located in the 3' region, compared to the mRNA produced mostly by SMN1. In addition, both genes have promoters with identical sequence, so it is believed that both are regulated similarly (Boda et al., 2004). The SMN1 gene is highly conserved on the evolutionary scale, with homologs found from single-cell organisms, such as the yeast Schizosaccharomyces pombe (Owen et al., 2000), to mammals (Schmid and DiDonato, 2007), although the SMN2 copy has only been found in hominids (Rochette et al., 2001). Therefore, since the gene is evolutionarily conserved, the mechanisms that regulate its expression or protein levels can be expected to be conserved as well.

Given that SMN2 produces a small amount of mRNA with exon 7 included, leading to only 10% of complete SMN protein (Jodelka et al., 2010), increasing the copy number of SMN2, or its splicing efficiency to allow the inclusion of exon 7 in the mRNA, have emerged as targets for use as a treatment for the disease. In fact, the first drug to appear against SMA, Nusinersen, was approved in 2016, which is an antisense oligonucleotide that specifically binds to a splicing silencer of SMN2 mRNA, thereby increasing the amount of processed mRNA that includes exon 7 (Messina, 2018). Moreover, there are currently two more drugs approved, and these drugs also target SMN genes (Mendell et al., 2017; Ratni et al., 2018).

The three treatments cited above are showing discrete results with the most severe types of SMA and have opened a hopeful stage in the treatment of the disease (Talbot and Tizzano, 2017). However, their effects need to be assessed in the longer term and when the disease is more advanced, something that is now beginning to be done with very uneven results, in addition to the high cost of these treatments (Chen et al., 2021; Coratti et al., 2021; Dangouloff et al., 2021; Pane et al., 2023). A new and promising strategy to treat SMA would be to increase the amount of SMN protein available in cells, either by increasing expression of the SMN2 gene, or by increasing the half-life of the mRNA and/or the protein itself, or even by increasing the localization of the protein in places in the cell where it is most needed. All this would be favored by the fact that the SMN protein is regulated by post-translational modifications, including phosphorylation, ubiquitination and sumoylation (Han et al., 2016; Husedzinovic et al., 2015; Navascues et al., 2008; Schilling et al., 2021), and these modifications regulate both its degradation and localization.

Different animal models have been used in the study of SMA, such as mouse, zebrafish, Drosophila and C. elegans. In all these models, advances in the knowledge of the smn gene have been described, related to the characterization of mutants, expression studies in different tissues, genetic interactions, confirmation of functions in conserved pathways and screening of potential drugs (Burghes et al., 2017; Konieczny and Artero, 2020; O'Hern et al., 2017). In the case of invertebrate organisms, despite their great evolutionary divergence from humans, it has recently been shown that the SMN complex is very well conserved and therefore these organisms are a good model to study SMA (Lanfranco et al., 2017). In the nematode C. elegans, RNAi knock-down of the human SMN1 orthologue, smn-1, leads to a decrease in egg laying, and surviving animals show developmental and neuromuscular deficiencies (Miguel-Aliaga et al., 1999). The smn-1(ok355) null mutation produces early death. Therefore, it is necessary to balance the mutation with a copy of the wild-type gene (Briese et al., 2009) or using point mutations in the smn-1 gene to avoid lethality (Sleigh et al., 2010). Currently, there are initiatives to develop animal models that allow drug screening, in which drug effect, clinical benefit and toxicity can be assessed (O'Hern et al. 2017), or new drugs can be tested (Konieczny and Artero, 2020). None of these models propose a strategy that allows easy and rapid measurement of the amount of SMN protein in the organism.

In this invention, we developed a novel system for drug repositioning which would act on putative SMN modifiers. First, we have generated a C. elegans strain with the fluorescent protein mCherry fused to the smn-1 protein using CRISPR technology where we can easily observe the localization and levels of SMN protein in vivo. Then, we have selected a list of new putative SMN modifiers proposed by a computational mass screening strategy based on the construction of gene expression profiles from public transcriptomic experiments. Next, we inhibited the candidate genes by RNAi in the strain carrying the mCherry-tagged SMN. For those candidates that increase SMN levels we identified inhibitory drugs and tested them to see if they generate the same effect as RNAi inhibition. Finally, both the most promising gene and drug were tested in cell cultures of SMA patients.

Following this strategy, we discovered and described a new SMN modifier gene, SMPD1, whose inhibition shows an increase in the expression of SMN genes and protein. This gene is involved in a sphingolipid metabolism pathway associated with a number of neurodegenerative diseases (Alessenko and Albi, 2020). Although imbalances in lipid metabolism have previously been found in SMA (Deguise et al., 2019a), this disease has never before been linked to sphingolipid metabolism. We also identified SMPD1 inhibitor drugs used in humans for other diseases that can be repurposed to treat SMA. The strategy presented here represents not only a new therapeutic approach for this disease, but also a new way to study its molecular basis.

### BRIEF DESCRIPTION OF THE FIGURES

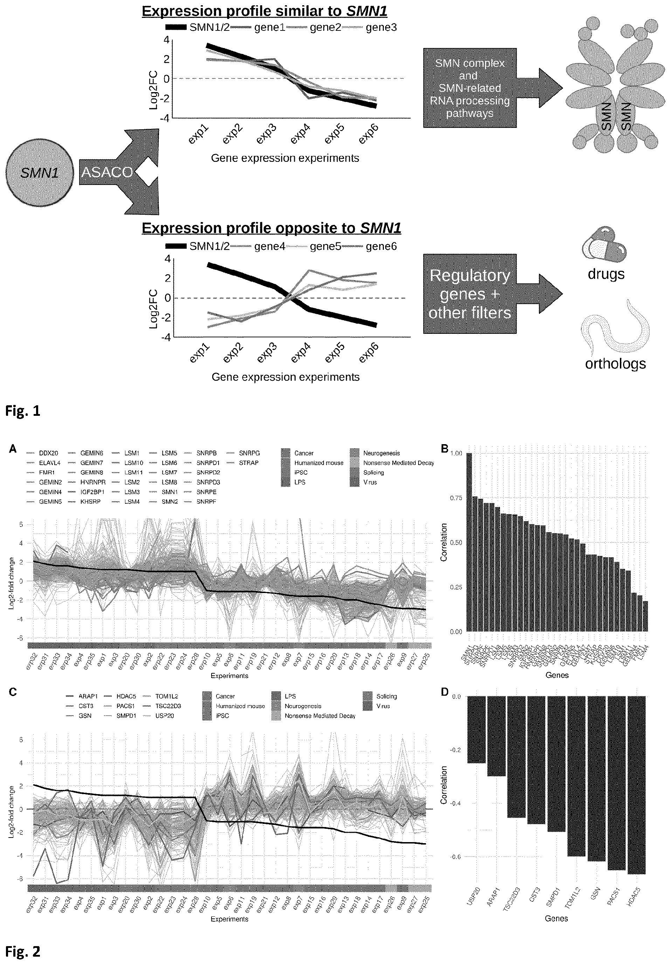

**Fig. 1****.** Protocol followed for the search for genes with similar or opposite expression profiles to SMN1 genes. The ASACO algorithm is used to search for positive correlators, which should include genes from the SMN complex and other RNA processing pathways involving SMN. Subsequently, negative correlators can be searched for, i.e. genes whose inhibition can be associated with an increase in SMN1 expression (and vice versa). Then, we can search for regulatory genes, with the idea of discovering negative regulators. We can also manually filter the results to keep only genes that have homologs in a given model organism. Finally, ASACO can search for inhibitory drugs that have the same effect as the negative correlators found.
**Fig. 2****.** Positive and negative SMN1 correlators obtained with ASACO. (A) Expression profiles found with ASACO for the SMN complex and related pathways, together with the expression profile of SMN1, across 34 differential expression experiments from the Expression Atlas database. The experiments have been classified according to the general context of the experiment performed. The gray lines correspond to other positive correlators. (B) Pearson correlation values between the expression profiles of the genes that are part of the SMN complex and related pathways versus the expression profile of the SMN1 gene. (C) Expression profiles of the candidate negative modifiers found with ASACO, together with the expression profile of SMN1, throughout the same 34 differential expression experiments. The gray lines correspond to other negative correlators not finally selected as candidates (199 genes). (D) Pearson correlation values between the expression profiles of the candidate negative modifiers found with ASACO versus the expression profile of the SMN1 gene.

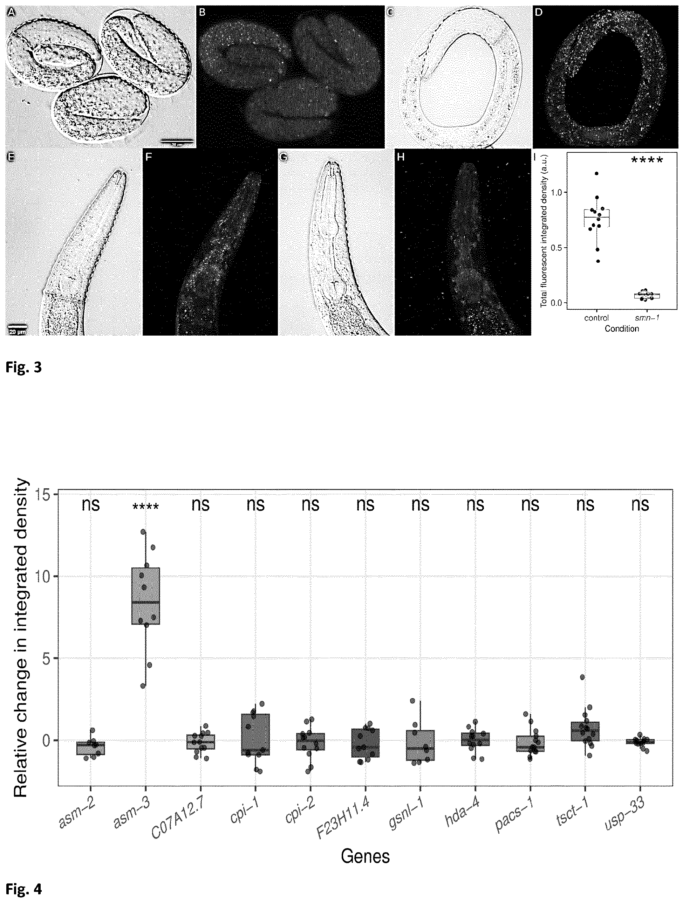

**Fig 3****.** Microscopy images of nematode strain mCherry::smn1 and fluorescence quantification. (A) Embryos under visible light and (B) under UV. Note that fluorescence is found in all embryo tissues and especially in the head (white dashed line). (C) First larval stage (L1) under visible light and (D) UV light. Note that fluorescence is again mainly located in the head of the nematode, where most of the neurons are present as also along the animal compatible with being expressed in the nerve cord. (E) Representative picture of the head of a L4 nematode under visible and (F) UV light. (G) Nematodes treated with RNAi of smn-1 under visible and (H) UV light. (I) Quantification of the fluorescence level of untreated nematodes (control) vs treated with smn-1 RNAi (t-test, p = 1.2e-07).
**Fig. 4****.** Relative level of SMN-1 after RNAi treatment for the 11 candidate genes. The level of fluorescence of the head of approximately 10 RNAi treated animals for each gene were measured, and compared to the fluorescence level of 10 non-treated animals. Each control is performed the same day and condition as the experiment. 0 value in the relative change of expression indicates no changes where positive value means increased level in the candidate clone. The experiment was repeated at least two times more with similar results (Suppl. Fig. S1). The name of the human homologue gene of these genes can be found in Table 1. Note that the RNAi of asm-3 generates an increase of the level of SMN-1 compared to control with no RNAi (Benferroni t-test, p = 5.29e-6).

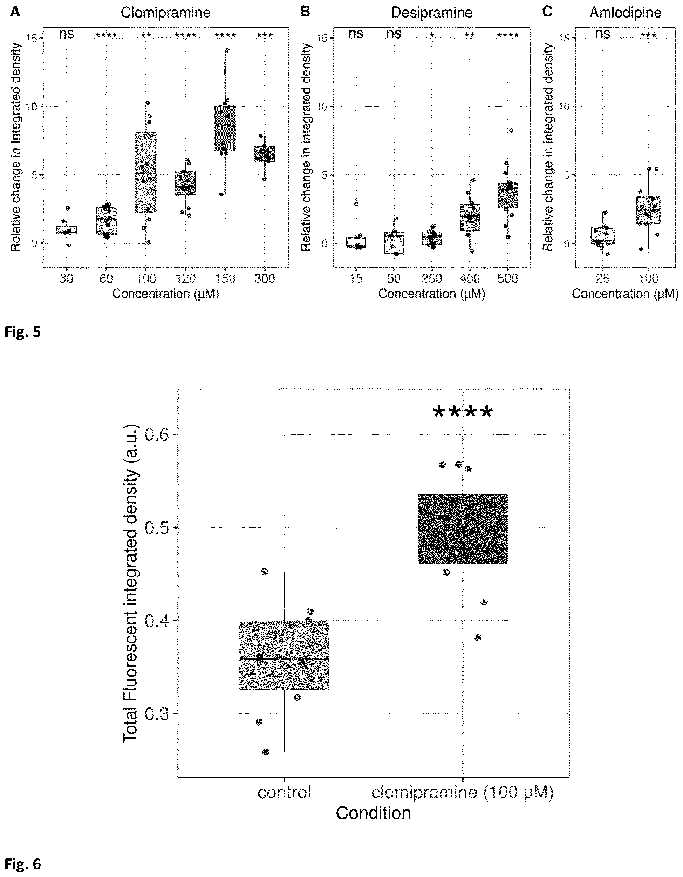

**Fig 5****.** SMN-1 levels increase after treatment with SMPD1 inhibitor drugs. The fluorescence level was measured in the heads of several animals for each concentration, and compared to the fluorescence level of non-treated animals. Each control was performed the same day and in the same conditions as the experiment. A value of 0 in the relative change in expression indicates no change and a positive value means an increase in the level with the treatment. The experiments were repeated with similar results (Suppl. Fig. S2). (A) Treatment with Clomipramine generated a significant increase in the levels of SMN-1 at 60 µM and above (B) Desipramine generated a significant increase in the levels of SMN-1 at 250 µM and above, and (C) Amlodipine at 100 µM. Benferroni t-test: from *p<1e-2 to ****p<1e-5.
**Fig. 6****.** The level of SMN increases after treatment with clomipramine exclusively during the adult stage. The level of fluorescence in the heads of approximately 10 animals per condition was measured. Total fluorescence in arbitrary units is represented. The nematodes were grown to adulthood and then treated during 48 hours with 100 µM clomipramine. The fluorescence level of the mCherry:smn-1 nematodes without any treatment is indicated as control. T-test, p = 8.5e-05.

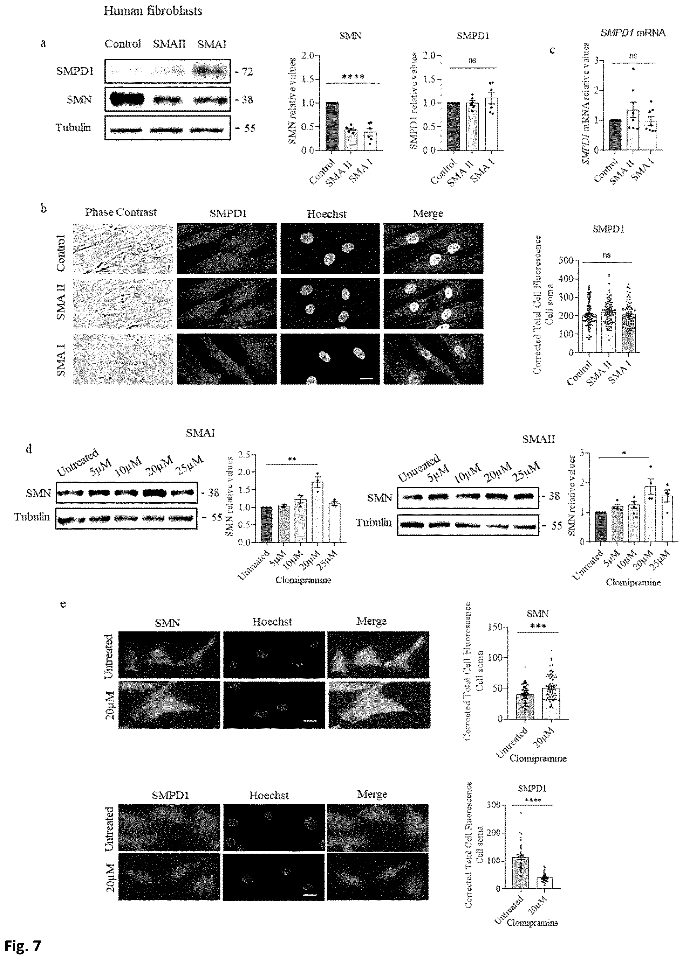

**Fig. 7****.** Clomipramine treatment increases SMN protein level in SMA fibroblasts. Control (unaffected) and SMAII and SMAI patient fibroblasts were plated in supplemented MEM medium and cultured for 48 h (a) Cell lysates were obtained and submitted to western blot using anti-SMPD1 and anti-SMN antibodies. Membranes were reprobed with anti-α-tubulin antibody. Graphs represent the expression of SMN or SMPD1 versus α-tubulin and correspond to the quantification of six independent experiments ± SEM. Asterisks indicate significant differences using one-way ANOVA with Tukey's multiple comparisons post-test (****p<0.0001; ns, no significant differences) (b) Representative immunofluorescence confocal images of SMAII human fibroblasts using anti-SMPD1 (red) antibody. Hoechst staining (blue) was used to identify cell nucleus. Graph represents the mean of relative SMPD1 fluorescence measured in cell soma, corresponding to the quantification of 80-110 cells per condition from 3 independent experiments ± SEM. Scale bar, 25µm (c) Total RNA was extracted and reverse transcribed to cDNA, Gapdh gene was used as control. Graph values are the mean of SMPD1 gene expression from eight independent experiments ± SEM (ns, no significant differences, p>0.05) (d) Forty-eight h cultured SMAII (left) and SMAI (right) fibroblasts were treated with 5µM, 10µM, 20µM, or 25µM clomipramine during 24 h or left untreated. Cell lysates were obtained and submitted to western blot using an anti-SMN antibody. Membranes were reprobed with an anti-α-tubulin antibody. Graphs represent the expression of SMN versus α-tubulin and correspond to the quantification of three or four independent experiments ± SEM. Asterisks indicate differences using one-way Anova with Tukey's multiple comparisons post-test (*p<0.05; **p<0.01) (e) SMAII fibroblasts were treated with 20µM clomipramine for 24 h or left untreated. Cultures were fixed and processed for immunofluorescence using anti-SMN (green) or anti-SMPD1 (red) antibodies. Hoechst (blue) dye was used to identify nuclei. Scale bar, 25µm. Graphs represent the mean of relative SMN or SMPD1 fluorescence measured in cell soma, corresponding to the quantification of at least 80 cells per condition from three independent experiments ± SEM. Asterisks indicate differences using Student t-test (****p<0.0001).

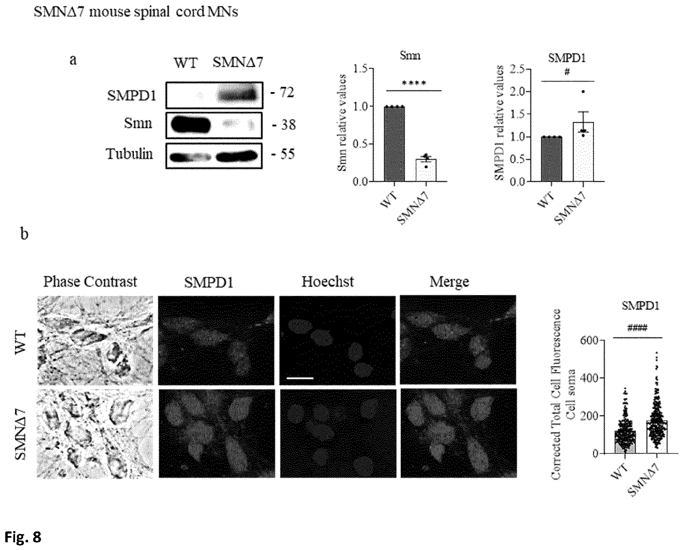

**Fig. 8****.** SMPD1 protein level in cultured mouse SMNΔ7 MNs. WT and SMNΔ7 spinal cord MNs were isolated and cultured in the presence of neurotrophic factors. After 6 days in vitro, total cell lysates were obtained (a) or cells were fixed for immunofluorescence protocol (b) (a) Protein extracts were submitted to western blot using anti-SMPD1 and anti-SMN antibodies. Membranes were reprobed with an anti-α-tubulin antibody, used as loading control. Graphs represent the expression of SMN or SMPD1 corresponding to the quantification of four independent experiments ± SEM. Symbols indicate differences using Student t-test (****p<0.0001) or the non-parametric Mann-Whitney test (#p<0.05) (b) Representative confocal immunofluorescence images of 6-day cultured WT and SMNΔ7 MNs using an anti-SMPD1 antibody. Hoechst (blue) staining was used to identify cell nucleus. Scale bar, 20µm. Graph represents the mean of relative SMPD1 fluorescence measured in cell soma, corresponding to the quantification of at least 250 cells per condition from three independent experiments ± SEM. Symbol indicates differences using non-parametric Mann-Whitney test (####p<0.0001).

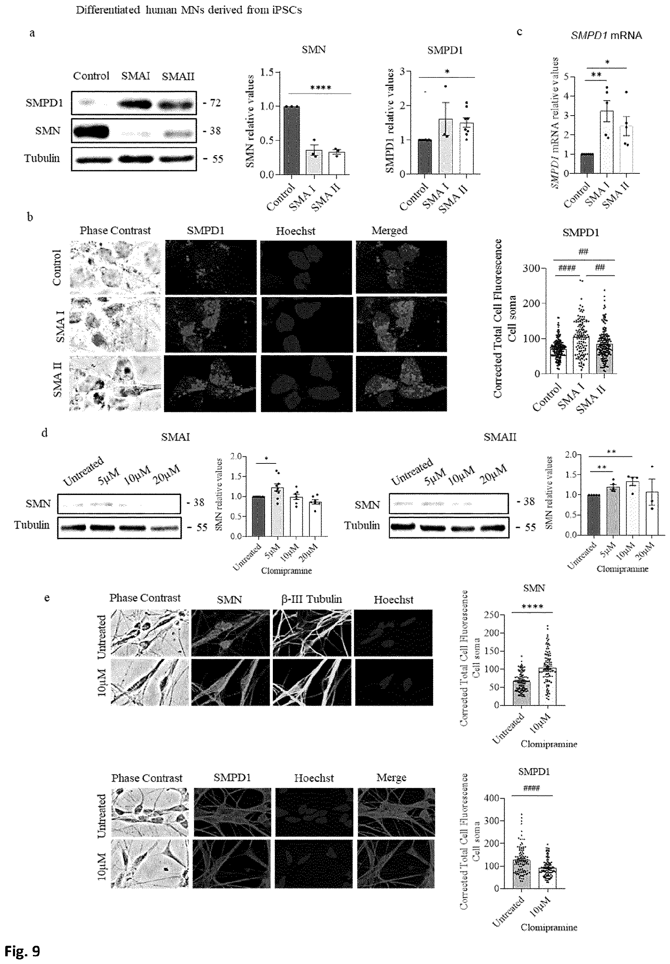

**Fig. 9****.** Clomipramine treatment increases SMN in differentiated human SMA MNs. Control, SMAI and SMAII MNs were differentiated for 6 days (a) Protein extracts were obtained and submitted to western blot analysis using anti-SMPD1 and anti-SMN antibodies, and membranes were reprobed with an anti-α-tubulin antibody. Graphs represent the expression of SMN or SMPD1 versus α-tubulin, corresponding to the quantification of at least 3 independent iPSCs differentiation cycles ± SEM. Asterisks indicate differences using one-way ANOVA (****p<0.0001; *p<0.05) (b) Representative phase contrast (left panels) and immunofluorescence images of cultured human MNs using an anti-SMPD1 antibody (red). Hoechst staining (blue) was used to identify MN nucleus. Graph values represent the mean of relative SMPD1 fluorescence measured in cell soma corresponding to the quantification of at least 130 cells per condition from 3 independent iPSCs differentiation cycles ± SEM. Symbols indicate differences using non-parametric Kruskal-Wallis test with Dunn's multiple comparisons post-test (####p< 0.0001, ##p< 0.005). Scale bar, 20µm (c) Total RNA was extracted from 6-day differentiated Control, SMAI and SMAII cells and reverse transcribed to cDNA. Gapdh gene was used as a control. Graph values are the mean of SMPD1 mRNA from 5 independent iPSCs differentiation cycles ± SEM. Asterisks indicate differences using one-way ANOVA with Bonferroni's multiple comparisons post-test (**p<0.005, *p<0.05) (d) Six-day differentiated human SMAI (left) and SMAII (right) MNs were treated with 5µM, 10µM, or 20µM clomipramine during 24 h. Cell lysates were obtained and submitted to western blot using anti-SMN antibody. Membranes were reprobed with an anti-α-tubulin antibody. Graphs represent the expression of SMN versus α-tubulin and correspond to the quantification of at least 3 independent iPSCs differentiation cycles ± SEM. Asterisks indicate differences using Student t-test (*p<0.05, **p<0.01) (e) Differentiated SMAI MNs were treated with 10µM clomipramine for 24 h. Cultures were fixed and processed for immunofluorescence using anti-SMN (green), anti-SMPD1 (red), or anti-β-III Tubulin (yellow) antibodies. Hoechst (blue) dye was used to identify cell nucleus. Graphs represent the mean of relative SMN or SMPD1 fluorescence measured in cell soma, corresponding to the quantification of at least 100 cells per condition from 3 independent differentiation cycles ± SEM. Symbols indicate differences using Student t-test (****p<0.0001) or non-parametric Mann-Whitney test (####p<0.0001). Scale bar, 40µm.

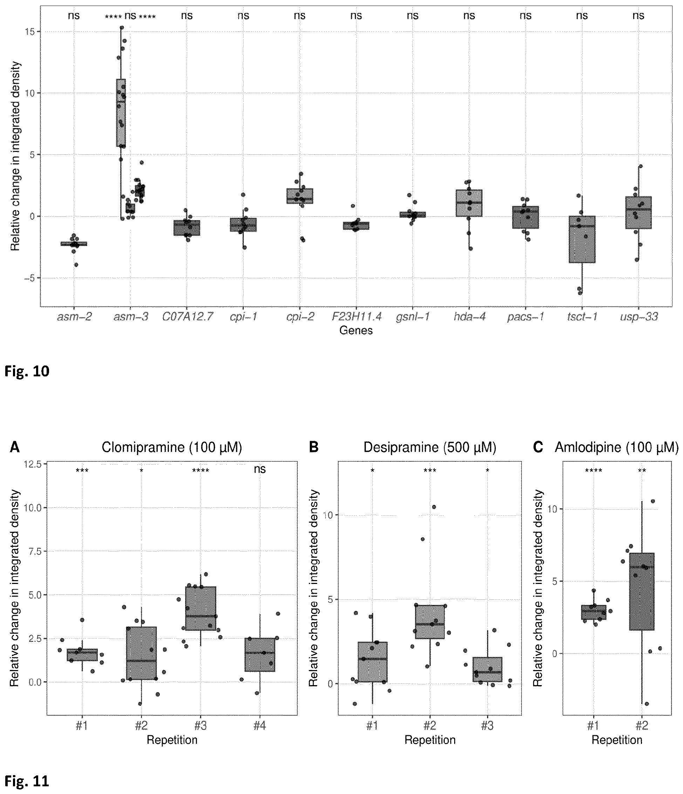

**Fig. 10****.** Standardized change in expression of smn-1 after RNAi treatment of the 11 candidate genes. The level of fluorescence in the heads of several RNAi-treated animals for each gene was measured, and compared to the fluorescence level of 10 non-treated animals. Each control was performed the same day and condition as the experiment. 0 value in the relative change of expression indicates no changes where positive value means increased level in the candidate clone. The colors indicate the common day of the experiment. The experiment was repeated at least two times more with similar results (Fig. 4). The name of the human homologue gene of these genes can be found in Table 1. Note that the RNAi of asm-3 generates an increase of the level of SMN compared to control with no RNAi in two of the three experiments. Benferroni t-test: from *p<1e-2 to ****p<1e-5.
**Fig. 11****.** Treatment with SMPD-1 inhibitors increases the level of SMN-1. The fluorescence level was measured in the heads of several animals for each concentration, and compared to the fluorescence level of non-treated animals. Each control was performed the same day and in the same conditions as the experiment. A value of 0 in the relative change in expression indicates no change and a positive value means an increase in the level with the treatment. (A) Several experiments performed on different days with Clomipramine at 100 µM. (B) Several experiments performed on different days of treatment with Desipramine at 500 µM. (C) Several experiments performed on different days with Amilodipine at 100 µM. Notice that in all the cases the treatment generates a statistically significant increase of the level of SMN-1. Benferroni t-test: from *p<1e-2 to ****p<1e-5.

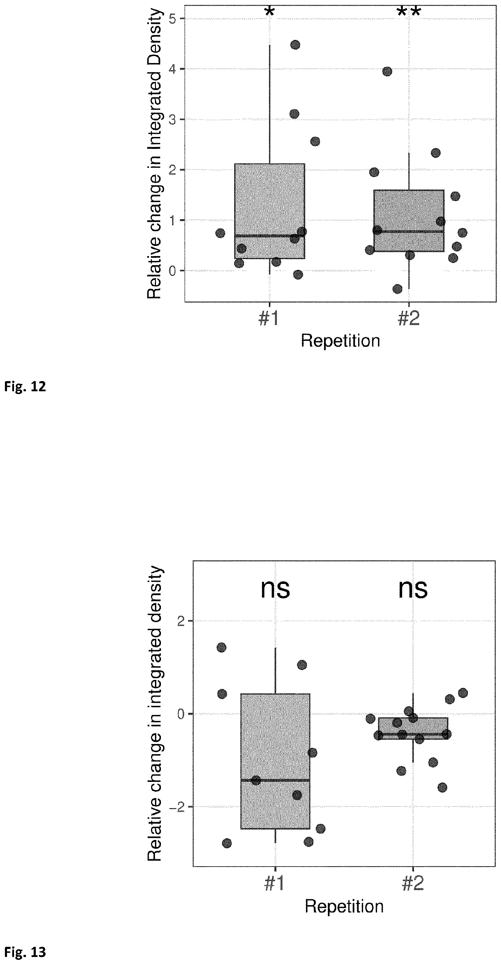

**Fig. 12****.** smn-1 expression after treatment of 100 µM of clomipramine exclusively during the adult stage. Two different assays showing treatment with 100 µM of clomipramine during 48 hours. The treatment started when animals reached adulthood. The fluorescence level was measured in the heads of at least 10 animals in each repetition, and compared to the fluorescence level of non-treated animals. Each control was performed the same day and in the same conditions as the experiment. A value of 0 in the relative change in expression indicates no change and a positive value means an increase in the level with the treatment. Notice that in both repetitions there is a statistically significant increase of the levels of SMN-1. Benferroni t-test: from *p<1e-2 to ****p<1e-5.
**Fig. 13****.** mCherry fluorescence level is not affected with 100 µM of clomipramine. To test if the changes in the level of fluorescence observed upon clomipramine treatment is due to changes in mCherry fluorescence or stability, we performed the treatment in the strain BN1158 which expresses the mCherry non-fused to SMN-1. No changes in the fluorescence levels werw observed in any of the repetition of the experiment. The fluorescence level was measured in the heads of approximately 10 animals in each repetition, and compared to the fluorescence level of non-treated animals. Each control was performed the same day and in the same conditions as the experiment. A value of 0 in the relative change in expression indicates no change and a positive value means an increase in the level with the treatment. ns means no statistically significant differences were observed. Benferroni t-test: from *p<1e-2 to ****p<1e-5.

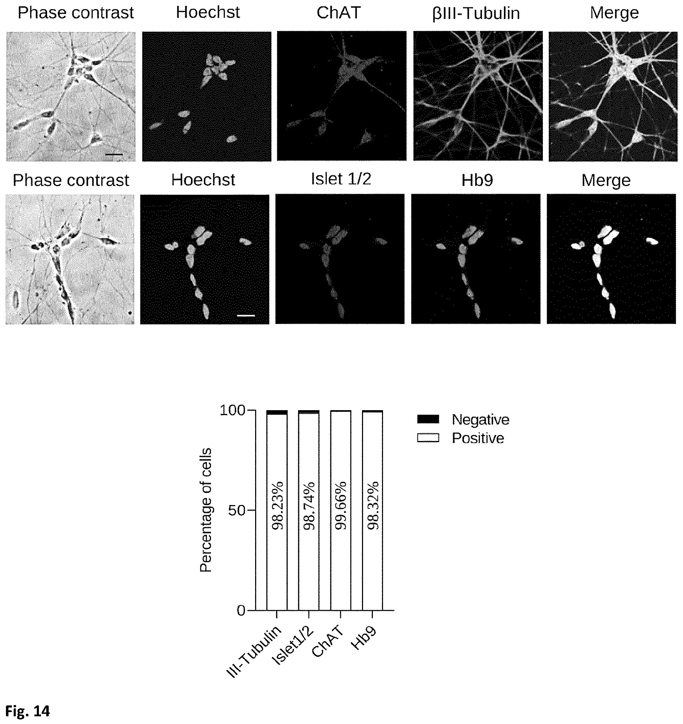

**Fig. 14****.** MN markers expressed in differentiated human iPSCs. Representative phase contrast and immunofluorescence images of 7-day differentiated human SMAI cells stained against the MN markers: choline acetyltransferase (ChAT, red, top panel), Islet1/2 (red, bottom panel), and Hb9 (green, bottom panel); and the neuronal indicator βIIITubulin (green, top panel), using anti-ChAT (1: 100, Cat. No. ab18736, Abcam, Cambridge, UK), anti-lslet1/2 (1: 50, Cat. No. 39.4D5, Developmental Studies Hybridoma Bank, Iowa City, IA, USA), anti-Hb9 (1: 75, Cat. No. ab92606, Abcam), and anti-βIIITubulin (1:400, Cat. No. 5568, Cell Signaling Technology, Danvers, MA, USA) antibodies, respectively. Hoechst staining (Sigma) (blue) was used to identify MN nucleus. Graph represents the percentage of βIIITubulin, Islet1/2, ChAT and Hb9 positive (98.23%, 98.74%, 99.66%, and 98.32%, respectively) and negative (1.76%, 1.26%, 0.34%, and 0.68%, respectively) cells. Data are presented as mean of two independent differentiation cycles of SMAI iPSCs. Scale bar, 20µm.

### DESCRIPTION OF EMBODIMENTS

Progress in understanding rare diseases is often slow, due to the small number of patients who suffer from them. The objective is usually oriented towards the search for treatments, which take time to develop and are expensive for patients (Connock et al., 2020). Therefore, drug repositioning is becoming a widely used procedure in this field, due to the ease of implementation of the entire screening process, and the low cost of the final product found (Chong et al., 2021; Konieczny and Artero, 2020).

In the present invention, we herein show a multidisciplinary protocol starting with the search for gene modifiers linked to Spinal Muscular Atrophy. These genes have been found using a computational algorithm, which is based on expression profiles throughout public transcriptomics experiments. This protocol would allow the study of any rare disease, even if there were no specific gene expression experiments associated with it.

Subsequently, we used a rapid screening system, based on a C. elegans strain in which the protein of interest has been fluorescently tagged. This strain allows us to test whether inhibition of the candidate genes generates an increase in SMN protein levels. And once the candidates are experimentally validated, we can move on to the demonstration with human patient cells. Following this protocol, we have found 9 candidates, of which one, SMPD1, has given successful results both in C. elegans and in cell cultures.

SMPD1 is a sphingomyelin phosphodiesterase that converts sphingomyelin to ceramide and whose mutations cause Niemann-Pick disease, a metabolic disease that leads to the accumulation of sphingolipids in different parts of the body and leads to progressive neurodegeneration in its most severe cases (Jones et al., 2008; Schuchman et al., 1991). In fact, ceramide accumulation can lead to neuronal apoptosis (Toman et al., 2002).

We have found significant overexpression of SMPD1 in SMA patient motor neurons. Sphingolipids are essential lipids for membrane stability and signaling pathways and they are mediators in neurodegeneration (McCluskey et al., 2022). Although disorders in sphingolipid metabolism have been associated with many neurodegenerative diseases, it has never before been associated with SMA.

In the case of SMA, although the SMN protein has pleiotropic functions, the main phenotype observed is the motor neuron degeneration (Tisdale and Pellizzoni, 2017). So if this phenotype is caused by the presence of high levels of the SMPD1 enzyme, the simple inhibition of this enzyme could be sufficient to slow down the progression of the disease.

Some of the ceramide-regulated pathways are also altered in human MNs models of SMA, such as decreased activity of PI3K/Akt pathway (Branchu et al., 2013; Sansa et al., 2021), increased activity of JNK pathway and apoptosis (Genabai et al., 2015; Schellino et al., 2018), and alterations in cell membrane fluidity (Schmitt et al., 2014). In addition, a low-fat diet has been shown to increase the survival of murine models of SMA, which may help to prevent ceramide from accumulating (Deguise et al., 2019b). These precedents raise the question of whether the imbalance in sphingolipid metabolism, of particular importance in neuronal membranes, might help to understand why SMA is a disease of primarily neuronal involvement, despite the fact that the SMN protein is mainly involved in different RNA pathways, which has been debated and unresolved since the SMN1 gene was first associated with the disease (Burghes and Beattie, 2009).

Our screening protocol finally allows us to search for drugs that can inhibit the modifier gene. We found that tricyclic antidepressants are well known to inhibit acid sphingomyelinase activity (Hurwitz et al., 1994), and it may act at the transcriptional level (Rhein et al., 2021). In addition, it has been shown that in mouse hippocampus, the use of these kind of drugs decreases the concentration of ceramide and increases neuronal proliferation (Gulbins et al., 2013). Thus, we have found that at least one of the drugs in this group, clomipramine, reduces acid sphingomyelinase expression in human SMA fibroblasts and MNs. This drug, as well as being used as an antidepressant, has been shown to reduce symptoms in diseases such as Multiple Sclerosis in a mouse model (Faissner et al., 2017) or in clinical trials with Huntington's disease (Achenbach et al., 2021). We now propose this drug, as well as its chemical derivatives, as a possible new treatment against SMA, because it produces an increase in the levels of SMN protein. This is something that has not been previously described, and that could represent a new treatment for this disease, alone or in combination with other already approved drugs.

Although ceramide levels have not been analyzed in this invention, the results obtained suggest ceramide as a possible marker to measure disease progression as well as the evolution after the use of anti-disease drugs, as has been shown with sphingomyelinase-rich exosomal structures in cerebrospinal fluid in multiple sclerosis (Pieragostino et al., 2018).

In conclusion, we have presented a multidisciplinary protocol capable of proposing gene modifying, and ultimately inhibitory drugs, for use as treatment in understudied diseases, showing novel and promising results for SMA patients.

Therefore, a first aspect of the invention refers to a composition comprising an inhibitory molecule capable of inhibiting the activity or expression of a gene selected from any of the list consisting of SMPD1, ARAP1, CST3, GSN, HDAC5, PACS1, TOM1L2, TSC22D3 and USP20 or homologues thereof and increasing the levels of endogenous SMN1 and/or SMN2 proteins, or a mixture of two or more of said molecules, for use in the treatment of Spinal Muscular Atrophy (SMA) in a subject in need thereof.

In a preferred embodiment of the first aspect of the invention, the molecule is an acid sphingomyelinase (ASM) expression or activity inhibitor. The present invention provides a composition for preventing or treating degenerative SMA, including an ASM (acid sphingomyelinase) activity inhibitor or expression inhibitor as an active ingredient. The composition includes a pharmaceutical composition, optionally further comprising a carrier and/or one or more pharmaceutically acceptable excipients, or a food composition.

In the present invention, ASM (acid sphingomyelinase, gene Smpd1) is not limited to a specific biological origin. The amino acid sequence of ASM is not particularly limited to a specific amino acid sequence as long as it has an amino acid sequence of a polypeptide known as ASM, while the nucleic acid sequence of a gene encoding ASM (including all transcripts from ASM gene, such as mRNA and the like) is also not particularly limited. As a preferable example, human ASM may comprise an amino acid sequence known as Genbank Accession No. NP_000534.3, NP_001007594.2, NP_001305016.1, NP_001305017.1, and the like, while its gene (or mRNA) sequence is known as Genbank Accession No. NP_000543.4, NP_001007593.2, NP_001318087.1, NP_001318088.1, and the like. More preferably, the gene (or mRNA) sequence of ASM (acid sphingomyelinase, gene Smpd1) can be obtained in NCBI Gene ID 6609 (https://www.ncbi.nlm.nih.gov/gene/6609).

The ASM activity inhibitor according to the present invention may be at least one selected from a group consisting of a compound such as a tricyclic antidepressants (TCAs), a peptide, a peptide mimetic, a substrate analogue, an aptamer, and an antibody, specifically binding to ASM protein, but is not limited thereto. In a preferred embodiment, the ASMase inhibitor is selected from the group consisting of a compound selected from the list consisting of clomipramine, desipramine, amitriptyline, amlodipine, astemizole, benzatropine, citalopram, clomiphene, cloperastine, cyclobenzaprine, cyproheptadine, diphenhydramine, fexofenadine, fluoxetine, maprotiline, nortriptyline, paroxetine, promethazine, sertraline, and zoledronic acid. More preferably, the compound is selected from the list consisting of Clomipramine, Desipramine and Amlodipine.

According to the present invention, the peptide mimetics inhibit the binding domain of ASM protein, thus inhibiting the activity of ASM protein. The peptide mimetics may be peptides or non-peptides and may include amino acids linked by non-peptide bonds such as psi bonds (Benkirane, N., et al. J. Biol. Chem., 271:33218-33224, 1996). Moreover, the peptide mimetics may be "conformationally constrained" peptides, cyclic mimetics, or cyclic mimetics including at least one exocyclic domain, a link moiety (linking amino acid) and an active region. The peptide mimetics are constructed to resemble secondary structural features of Ubiquitin-Associated Protein 2 (UBAP2) and may mimic inhibitory features of macro molecules such as antibody (Park, B. W. et al. Nat Biotechnol 18, 194-198, 2000) or water-soluble receptors (Takasaki, W. et al. Nat Biotechnol 15, 1266-1270, 1997). These peptides represent small molecules that may act with potency equivalent to the natural antagonist (Wrighton, N. C. et al. Nat Biotechnol 15, 1261-1265, 1997).

The aptamer is a single-stranded DNA or RNA molecule and may be obtained by isolating oligomers that bind to specific chemical molecules or biological molecules with high affinity and specificity by an evolutionary method using an oligonucleotide library called systematic evolution of ligands by exponential enrichment (SELEX) (C. Tuerand L. Gold, Science 249, 505-510, 2005; A. D. Ellington and J. W. Szostak, Nature 346, 818-822, 1990; M. Famulok, et. al., Acc. Chem. Res. 33, 591-599, 2000; D. S. Wilson and Szostak, Annu. Rev. Biochem. 68, 611-647, 1999). The aptamer may specifically bind to a target to regulate its activity and may inhibit the function of the target by binding, for example.

The antibody specifically and directly binds to the ASM to effectively inhibit its activity. Preferably, a polyclonal antibody or monoclonal antibody may be used as the antibody that specifically binds to the ASM. The antibody that specifically binds to the ASM may be prepared by a method known to those skilled in the art, and a commercially available ASM antibody may be purchased and used. The antibody may be prepared by injecting the ASM protein as an immunogen into an external host according to a conventional method known to those skilled in the art. The external host may include mammals such as mice, rats, sheep, rabbits, etc. The immunogen may be injected intramuscularly, intraperitoneally, or subcutaneously, and generally may be injected with an adjuvant to enhance antigenicity. Blood samples may be taken from the external host at regular intervals and serum exhibiting titer and specificity to the antigen may be collected to separate an antibody therefrom. It is noted that the term "antibody" includes antibody fragments.

The ASM expression inhibitor according to the present invention may be at least one selected from a group consisting of an antisense nucleotide, small hairpin RNA (shRNA), small interfering (siRNA), microRNA (miRNA) and ribozyme, complementarily binding to mRNA of an ASM gene or a gene promoting expression ofASM, but is not limited thereto. The antisense nucleotide, shRNA, siRNA or miRNA of the present invention is not particularly limited in its nucleotide sequence and its length as long as it has an activity of suppressing the expression of the ASM gene (i.e., Smpd1).

The siRNA is composed of a sense sequence of 15 to 30-mers selected from the mRNA sequence of a gene that encodes the ASM protein and an antisense sequence complementarily binding to the sense sequence. Here, preferably, the sense sequence may be composed of about 25 nucleotides, but is not particularly limited thereto.

As used herein, the miRNA (microRNA) refers to a short non-coding RNA derived from an endogenous gene, which acts as a post-transcriptional regulator of gene expression. miRNA acts as a post-transcriptional regulator of gene expression by forming a base pair with the mRNA of a target gene. The miRNA according to the present invention is not particularly limited in its nucleotide sequence and its length as long as it has an activity of suppressing the expression of the ASM gene (i.e., Smpd1).

As defined by Watson-Crick base pairs, the antisense nucleotide is hybridized with a complementary sequence of DNA, immature-mRNA or mature-mRNA to interrupt the transmission of genetic information as a protein in DNA. A target sequence specific antisense nucleotide is exceptionally multi-functional. The antisense nucleotide is a long chain of monomers, which favors hybridization to a target RNA sequence. Numbers of reports have recently been made to prove the utility of an antisense nucleotide as a biochemical tool in study of a target protein (Rothenberg et al., J. Natl. Cancer Inst., 81:1539-1544, 1999). Great progress has been made in the fields of oligonucleotide chemistry and nucleotide synthesis having improved cell adhesion of oligonucleotide, target binding affinity and resistance against nuclease, suggesting that an antisense nucleotide might be considered a new form of an inhibitor.

The composition of the present invention may include, together with the ASM activity inhibitor or expression inhibitor, at least one of a known active ingredient having an effect of inhibiting ASM expression or activity, or a known active ingredient having an effect of treating SMA.

Also, the pharmaceutical composition of the present invention may be administered orally or parenterally (e.g., applied intravenously, subcutaneously, intraperitoneally or topically) according to the intended use, and the dosage may vary according to a patient's weight, age, gender, health condition, diet, administration time, administration method, administration period or interval, excretion rate, constitutional specificity, nature of formulation, etc. The dosage of the ASM expression inhibitor or activity inhibitor of the present invention is about 0.001 to 1000 mg/kg per day, preferably about 0.1 to 500 mg/kg per day, but this may vary depending on the clinical test result. Preferably, the pharmaceutical composition of the present invention may be administered once or several times a day.

The pharmaceutical composition of the present invention may be formulated in a variety of formulations for administration. The excipients that may be included in the present invention are non-toxic inert pharmaceutically suitable solid, semi-solid or liquid formulation auxiliaries of any type, for example, fillers, weighting agents, binders, wetting agents, disintegrating agents, dispersing agents, surfactants or diluents, etc.

The pharmaceutical composition of the present invention may be formulated in the form of tablets, coated tablets, capsules, pills, granules, suppositories, solutions, suspensions and emulsions, pastes, ointments, gels, creams, lotions, powders or sprays.

The composition of the present invention may be added to dietary supplements for the improvement of SMA. When using the ASM expression inhibitor or activity inhibitor of the present invention as a food additive, the active ingredient may be added as it is or together with other food or food ingredients, and it may be suitably used according to a conventional manner. The amount of active ingredient added may be determined properly according to the purpose of use (preventive, health or therapeutic purposes). In general, when manufacturing food or beverage, the active ingredient of the present invention is added in an amount of 15% by weight or less to the raw material, preferably in an amount of 10% by weight or less. However, for health and hygiene purposes, or for long-term intake for the purpose of health control, the amount of active ingredient may be equal to or less than the above range, and since there is no problem in terms of safety, the active ingredient may be used in an amount greater than or equal to the above range.

There is no particular limitation in the type of food. Examples of the food to which this substance may be added include meat, sausages, bread, chocolate, candies, snacks, confectionery, pizza, ramen, other noodles, gum, dairy products including ice cream, soup, beverages, tea, drinks, alcohol drinks, and vitamin complexes, etc. That is, food may comprise all kinds of dietary supplements in the conventional sense.

The health beverage composition of the present invention may include additional ingredients such as various flavoring agents or natural carbohydrates, etc., like other beverages. The natural carbohydrates above may be monosaccharides such as glucose and fructose, disaccharides such as maltose and sucrose, polysaccharides such as dextrin and cyclodextrin, and sugar alcohols such as xylitol, sorbitol and erythritol, etc. Natural sweeteners such as thaumatin and stevia extract, and synthetic sweeteners such as saccharin and aspartame, etc. may be used as sweeteners. The ratio of natural carbohydrate is generally in the range of about 0.01 to 0.20 g per 100 g of the composition of the present invention, and preferably in the range of about 0.04 to 0.10 g.

In addition to the above, the composition of the present invention may include various nutrients, vitamins, electrolytes, flavoring agents, coloring agents, pectic acid and its salts, alginic acid and its salts, organic acid, protective colloidal thickeners, pH adjusting agents, stabilizers, preservatives, glycerin, alcohol, carbonating agents used in carbonated beverages, etc. Further, the composition of the present invention may include pulp for the production of natural fruit juice, fruit juice beverage and vegetable beverage. These ingredients may be used independently or in combination with other ingredients. The ratio of the additive is not so important but is generally selected from a range of 0.01 to 0.20 parts by weight with respect to 100 parts by weight of the composition of the present invention.

The term "comprising" of the present invention is used synonymously with "including" or "characterized" and does not exclude additional component elements or method steps, etc not mentioned in the composition or method.

The term "consisting of" is intended to exclude additional elements, steps or components, etc not otherwise mentioned. The term "essentially consisting of" is intended to encompass component elements or steps, etc., which, in addition to the described component elements or steps, do not materially affect the active ingredient (for instance, ASM inhibitor in the present invention) underlying properties in the scope of the composition or method.

Also, the present invention provides a method for preventing or treating SMA, including administering to a subject in need thereof a therapeutically effective amount of the composition.

Specifically, the present invention provides a method for treating SMA in a subject in need thereof, comprising administering to a subject a therapeutically effective amount of a composition comprising an ASM (acid sphingomyelinase) activity inhibitor or expression inhibitor as an active ingredient.

Also, the present invention provides a method for treating SMA in a subject in need thereof, comprising administering to a subject a therapeutically effective amount of a composition consisting of an ASM (acid sphingomyelinase) activity inhibitor or expression inhibitor.

Also, the present invention provides a method for treating SMA in a subject in need thereof, comprising administering to a subject a therapeutically effective amount of a composition essentially consisting of an ASM (acid sphingomyelinase) activity inhibitor or expression inhibitor.

Also, the present invention provides a method for the palliative treatment of one or more symptoms of Spinal Muscular Atrophy (SMA), comprising administering to a subject a therapeutically effective amount of a composition comprising, consisting of or consisting essentially consisting of an ASM (acid sphingomyelinase) activity inhibitor or expression inhibitor.

Also, the present invention provides a method for the palliative treatment of one or more symptoms of Spinal Muscular Atrophy (SMA), comprising administering to a subject a therapeutically effective amount of a composition comprising, consisting of or consisting essentially consisting of an ASM (acid sphingomyelinase) activity inhibitor or expression inhibitor.

The term "treatment or treating" as used herein is a concept involving inhibition, elimination, alleviation, relief, amelioration and/or prevention of a disease itself, or symptoms or conditions caused by the disease.

The "effective amount" as used herein refers to an amount that, when administered to an individual, represents an improvement, treatment, or prevention effect of SMA. It is obvious to those skilled in the art that the therapeutically effective amount may be determined within the scope of sound medical judgment. Preferably, the specific therapeutically effective amount for a particular patient may vary depending on a variety of factors including the type and degree of a desired reaction, the specific composition including the use of any other agents according to the intended use, the patient's age, weight, general health condition, gender, diet, administration time, administrate route and excretion rate of the composition, duration of treatment, other drugs used in combination or coincidentally with the specific composition, and like factors well known in the medical arts. Therefore, preferably, the effective amount of the composition suitable for the purpose of the present invention is determined in consideration of the foregoing.

In addition, optionally, the composition of the present invention may be administered in combination with a known therapeutic agent for SMA to increase the effect of treating SMA.

The term "subject" refers to an animal, preferably a mammal which especially includes a human, while including animal-derived cells, tissues, organs and the like. The subject may be a patient in need of the above mentioned effect.

The present invention is applicable to any mammal, with SMA as described in the above-mentioned "subject". Here, the mammals include human, primates, and livestock animals such as cows, pigs, sheep, horses, dogs, cats, etc.

Also, the present invention provides a method for screening a substance for preventing or treating SMA, including 1) treating a biological sample with a candidate substance, and 2) measuring the change in expression amount of mRNA or protein of ASM (acid sphingomyelinase) from the biological sample in step 1).

The biological sample in the step 1) may include blood, urine, saliva or tissue, etc. of animals with SMA, but is not limited thereto.

The method for measuring the change in expression amount of mRNA in the step 2) includes reverse transcription polymerase chain reaction (RT-PCR), competitive RT-PCR, realtime RT-PCR, RNase protection assay (RPA), Northern blotting, and DNA chip, etc., but is not limited thereto.

The method for measuring the change in expression amount of protein in the step 2) includes Western blotting, enzyme linked immunosorbent assay (ELISA), radioimmunoassay (RIA), radioimmunodiffusion, Ouchterlony immunodiffusion, rocket immunoelectrophoresis, immunohistostaining, immunoprecipitation assay, complement fixation assay, fluorescence activated cell sorter (FACS) and protein chip, etc., but is not limited thereto.

Hereinafter, preferred Examples will be provided to facilitate understanding of the present invention. However, the following Examples are provided for better understanding of the present invention only, and the scope of the present invention is not limited by the following Examples.

### EXAMPLES

### Material and Methods

### Selection of candidate gene

Genes with similar and opposite expression profile to SMN1 and SMN2 were searched using the ASACO bioinformatics algorithm (Pérez-Pulido et al., 2021). For each gene, transcriptomics experiments were searched in the Expression Atlas database release 32 in which the gene appeared as differentially expressed (Fold change ≥ 1 and p-value ≤ 0.05). Then, genes with a similar expression profile were searched for throughout those experiments, as well as those with an opposite profile.

Genes with similar expression profiles, or positive correlators (Pearson correlation near to 1), are expected to be genes functionally associated with the starting gene. As known positive correlators, those that participate with SMN in the protein complex of the same name and other RNA processing pathways were selected (Tisdale and Pellizzoni, 2017, p. 7).

Genes with opposite expression profile, or negative correlators (Pearson correlation near to -1), were further selected for final candidates. Only regulatory genes were initially taken, looking for the term 'regulator' or similar in their functional annotations: GO terms, UniProt Keywords and Reactome pathways extracted from Biomart (Ensembl release 98, Human GRCh38.p13, C. elegans WBcel235) (Kasprzyk, 2011). Subsequently, we selected those genes that were not only negative correlators of SMN1 or SMN2 when using ASACO but were also negative correlators for 3 other genes of the SMN complex or related pathways. Finally, candidate genes were those of them that had orthologs in C. elegans, according to OrthoList 2 (Kim et al., 2018), and those for which inhibitory drugs are known according to the DrugBank database release 5.1.5 (Wishart et al., 2018).

### C. elegans culture and general methods

C. elegans strains were cultivated under standard conditions (Stiernagle, 2006). The strains used are as follows: PHX2635: smn-1(syb2635); BN1158: bqSi1152[pBN521(unc-119(+) eft-3p::Frt::mCh::his-58::Frt::UPRT)]II; bqSi577[pBN306(unc-119(+) myo-2p::GFP)] IV. unc-119(ed9)III . The Escherichia coli strains used were: OP50 and HT115.

The strain PHX2635: smn-1(syb2635) was generated by CRISPR technology, we introduced the coding sequence of the fluorescent protein m-Cherry upstream of the TAA stop codon of the C. elegans SMN1 homologous gene, smn-1, as indicated in the sequence:

…TGTTCAAAATGTCTCAAAGGGTGAAGAAGATAACATGGCAATTATTAAAGAGTTTATGCGTTTC
AAGGTGCATATGGAGGGATCTGTCAATGGGCATGAGTTTGAAATTGAAGGTGAAGGAGAAGGCC
GACCATATGAGGGAACACAAACCGCAAAACTAAAGgtaagtttaaacatatatatactaactaaccctgattatttaaattttcag
GTAACTAAAGGCGGACCATTACCATTCGCCTGGGACATCCTCTCTCCACAGTTCATGTATGGAAG
TAAAGCTTATGTTAAACATCCGGCAGATATACCAGATTATTTGAAACTTTCATTCCCGGAGGGTT
TTAAGTGGGAACGCGTAATGAATTTTGAAGACGGAGGAGTTGTTACAGTGACGCAAGACTCAAG
gtaagtttaaacagttcggtactaactaaccatacatatttaaattttcagCCTCCAAGATGGAGAATTTATTTATAAAGTCAAACT
TCGAGGAACGAATTTCCCCTCGGATGGACCTGTTATGCAGAAGAAGACTATGGGATGGGAAGCT
TCAAGTGAAAGAATGTACCCTGAAGACGGTGCTCTTAAGGGAGAGATTAAACAACGTCTTAAAT
TGAAAGATGGAGGACATTACGATGCTGAGgtaagtttaaacatgattttactaactaactaatctgatttaaattttcagGTGAAGA
CAACTTACAAAGCCAAAAAACCAGTTCAGCTGCCAGGAGCGTACAATGTTAATATTAAACTGGA
TATCACCTCCCACAACGAGGATTACACTATCGTTGAGCAATATGAAAGAGCTGAAGGGCGGCAC
TCGACAGGTGGCATGGATGAATTGTATAAGTAA…

Sequence underlain belong to the smn-1 sequence, the rest of the sequence corresponds to the mCherry sequence inserted in the genome, lowercase letters indicated introns including in the mCherry gene.

RNAi feeding was performed as described (Kamath and Ahringer, 2003). RNAi clones belong to the Aringher gene library were used: JA:C41G7.1 (smn-1), JA:T18D3.7 (tsct-1), JA:WO3G1.7 (asm-3), JA:C07A12.7 (C07A12.7), JA:C10E2.3 (hda-4), JA:K09A9.4 (usp-33), JA:R01B10.1 (cpi-2), JA:K08B4.6 (cpi-1), JA:K06A4.3 (gsnl-1), JA:F23H11.4 (F23H11.4), JA:T18H9.7 (pacs-1), JA:C41G7.1 (smn-1) and MV_SV: mv_ZK455.4 (asm-2) from Marc Vidal's gene library (Kim et al., 2005). For smn-1 expression assays, gravid hermaphrodites were transferred to empty plasmid pL4440 or RNAi plates, incubated at 20°C for 48 hours and the next generation was analyzed at the L4 stage. For the drug assay, NGM plates were prepared at the indicated concentrations and the respective vehicle was used as a control, then proceeded as in the RNAi assay.

### C. elegans image acquisition and analysis

L4 worms were mounted in a coverslip with a 2% agarose pad. C. elegans confocal images were acquired using a Nikon A1R microscope equipped with a 60x Plan Apo lens of Numerical Aperture 1.4. Twelve pictures were taken for each worm to be integrated in a Z image. The scan field of 1024x1024 resulted in a pixel width of 209 nm and depth of 500 nm. Laser intensity, gain and pixel scan speed remained constant along all sets of experiments.

For image analysis, we used FIJI software. The experimentalist drew manually a ROI containing between the second pharynx bulb and the mouth of the nematode. Within this area, mCherry expression was detected using an intensity threshold that remained constant along all images. This minimum intensity level was defined by the experimenter. Within the thresholded volume, we measured the integrated density using the plugin 3D object counter (Bolte and Cordelières, 2006). The CSV files were exported for subsequent statistical analyses using the R programming language. The statistical tests and thresholds used are shown in the figure captions.

### SMA animals

Experiments involved the SMA mice model FVB·Cg-Grm 7Tg(SMN2)89Ahmb Smn 1TM1Msd Tg(SMN2*delta7)4299Ahmb/J (SMNΔ7) (The Jackson Laboratory, Bar Har Harbor, Maine, USA). SMNΔ7 (Smn-/-; SMN2+/+; SMNΔ7+/+) mice were obtained by crossing heterozygous (Smn+/-; SMN2+/+; SMNΔ7+/+) animals. Littermates SMNΔ7 and WT (Smn+/+; SMN2+/+; SMNΔ7+/+) were used for the experiments.

To genotype the neonatal offspring, the Phire Tissue Direct PCR Master Mix (Thermo Fisher Scientific, Waltham, MA, USA) was used for genomic DNA extraction and PCR setup using the following primers: WT, forward 5' -CTCCGGGATATTGGGATTG-3'; SMA, reverse 5' - GGTAACGCCAGGGTTTTCC-3'; and WT, reverse 5' -TTTCTTCTGGCTGTGCCTTT-3'.

All procedures were approved by the University of Lleida Advisory Committee on Animal Services (CEEA02-02/23) and performed in accordance with the Spanish Council on Animal Care guidelines.

### Mouse motor neuron isolation and culture

Motor neuron (MN) primary cultures were obtained from the spinal cords of genotyped WT and SMNΔ7 E13 mouse embryos as previously described (Garcera et al., 2011; Gou-Fabregas et al., 2009). Isolated MNs were plated either in laminin-coated four-well tissue culture dishes (Nunc, Thermo Fisher Scientific) for western blot analysis (80,000 cells/well) or in laminin-coated glass coverslips placed in four-well dishes for immunofluorescence experiments (20,000 cells/well). MNs were maintained in neurobasal medium (Gibco, Thermo Fisher Scientific) supplemented with B27 (2% v/v) (Gibco), horse serum (2% v/v) (Gibco), L-glutamine (0.5mM) (Gibco), 2-mercaptoethanol (25µM) (Sigma, Saint Louis, MO, USA), and a cocktail of neurotrophic factors: BDNF (1ng/ml), GDNF (10ng/ml), CNTF (10ng/ml), and HGF (10ng/ml) (Peprotech, London, UK). Twenty-four hours after plating, 2µg/ml of aphidicolin (Sigma) was added to the culture medium and maintained during the course of the experiment.

### Human fibroblast cell lines culture

Human fibroblast cell lines were purchased to Coriell Institute for Medical Research (Camden, NJ, USA). The Coriell Cell Repository maintains the consent and privacy of the donor samples. Culture protocols in the present study were carried out under institutional review board guidelines at Universitat de Lleida and IRBLleida.

Two human fibroblasts cell lines from SMA patients (GM03813, SMA type 2 patient, SMAII; and GM09677, SMA type 1 patient, SMAI) and one healthy control (GM03814, Control) were obtained and cultured following the manufacturer instructions. Fibroblasts were maintained in Eagle's Minimum Essential Medium (MEM) (Sigma) supplemented with 15% (v/v) non-inactivated fetal bovine serum (FBS) (Gibco), 0.5M of L-Glutamine (Gibco), 1% (v/v) non-essential amino acids, and 20µg/ml of Penicillin-Streptomycin (Gibco). For western blot analysis, cells were plated at 3,000-4,000 cells/cm2 in 35mm dishes and cultured in supplemented MEM for 24 h. For immunofluorescence experiments, 3,000 cells/well were plated in collagen-coated 1cm2 glass coverslips placed into four-well dishes and fixed in 4% paraformaldehyde (Sigma) in PBS.

### Differentiation of human-induced pluripotent stem cell lines (iPSCs) to MNs

Three human iPSCs lines were used in the present work. The GM23411*B (Coriell Institute) iPSC cell line obtained from a healthy 3 months old individual was used as control. The GM23240*B (Coriell Institute) iPSCs obtained from a fibroblast cell line of a 3 years old SMA type 2 patient (2 copies *SMN2;* deletion exon7-8 in *SMN1*) was used as SMAII condition. SMAII patient was previously classified as SMA type 1, but several data supported re-classification to SMA type 2. The CS84iSMA-nxx (Cedar Sinai, Los Angeles, USA) iPSCs obtained from a lymphoblast cell line of a 6 months old SMA type 1 patient (2 copies *SMN2;* deletion exon7-8 in *SMN1*) was used as SMAI condition.

Human iPSCs were differentiated to MNs as previously described (de la Fuente et al., 2020; Du et al., 2015). Cell lines were cultured and expanded on Geltrex (Sigma)-coated plates in Essential 8 medium (Gibco). Neuroepithelial cells and MN progenitors were generated following the established protocol. To induce MN differentiation, MN progenitors were detached with Accutase (Gibco) and cultured in suspension in MN induction medium (DMEM/F12: NBM plus 1:1 supplemented with B27, L-glutamine [all from Gibco], 0.1mM ascorbic acid [Sigma], 3µM CHIR99021; 2µM SB431512, 2µM DMH1 [all from Cayman, Ann Harbor, MI, USA], 0.5µM retinoic acid [Sigma], and 0.1µM purmorphamine [Cayman]). After six days, neurospheres were dissociated with Accumax (Invitrogen, Waltham, MA, USA) and plated on laminin-coated plates in MN maturation medium: MN induction medium supplemented with 0.1µM Compound E (Sigma), 20ng/ml ciliary neurotrophic factor (CNTF) and 20ng/ml Insulin-like growth factor 1 (IGF-1) (both from Peprotech). Dissociated neurospheres were plated in laminin-coated four-well dishes for western blot analysis (90,000 cells/well), in 1cm² laminin-coated coverslips for immunofluorescence experiments (25,000 cells/well), or in laminin-coated six-well dishes (Falcon, Corning Incorporated, Corning, NY, USA) for RT-qPCR (500,000 cells/well).

### Western Blot analysis

Western blots were performed as previously described (Gou-Fabregas et al., 2009). Cell lysates were resolved in sodium dodecyl-sulfate polyacrylamide (SDS-PAGE) gels and transferred onto polyvinylidene difluoride (PVDF) Immobilon-P transfer membranes (Millipore, Burlington, MA, USA) using an Amersham Biosciences semidry Trans-Blot (Buckinghamshire, UK). Membranes were blotted with: anti-SMN (1: 5,000, Cat. No. 610646, BD Transduction Laboratories, Franklin Lakes, NJ, USA) and anti-SMPD1 (1: 300, Cat. No. 14609-1-AP, ProteinTech, Manchester, UK). To control the specific protein content per lane, membranes were reproved with anti-α-tubulin antibody (1: 50,000, Cat. No. T5168, Sigma). Blots were developed using Luminata^{™} Forte Western HRP Substrate (Millipore) and the ChemiDoc^{™} Imaging System (Bio-Rad, Hercules, CA, USA).

### Immunofluorescence

Cultured cells were fixed with 4% paraformaldehyde (Sigma) for 10 min and cold methanol (Sigma) for 30 min more. Cells were permeabilized with 0.3% Triton X-100 for 1 h and incubated with a solution of 0.3% Triton-X-100 with 10% BSA in PBS for 30 min. Primary antibodies (anti-SMN, 1: 5,000; anti-SMPD1 1: 50) were diluted in 0.3% Triton-X-100 and incubated overnight with 10% BSA in PBS. After washing, the secondary antibody was added: anti-mouse ALEXA555, (1: 400, Cat. No. A21422) and anti-rabbit ALEXA488 (1: 400, Cat. No. A11008) (both from Invitrogen, Waltham, MA, USA). Hoechst (1: 400, Sigma) staining was performed to identify nuclear localization in cultured cells. Microscopy observations were performed in a FV10i Olympus (Tokyo, Japan) confocal microscope. Quantification of fluorescence intensity was performed blinded, using the NIH ImageJ software (Schindelin et al., 2012). Using phase contrast images, the cell perimeter was manually delimited and transferred to the corresponding fluorescence images. The Integrated Density (IntDen) was measured in the selected area of the cell. Three non-fluorescent surrounding regions were measured to set up the background readings. The fluorescence intensity was calculated using the following formula: Corrected Total Cell Fluorescence (CTCF) = IntDen (sum of the values of the pixels in the selection) - (area of the cell x mean fluorescence of the background).

### RNA isolation and quantitative RT-PCR

For RT-qPCR experiments, cells (human fibroblasts and differentiated MNs) were plated at a density of 500,000 cells/well in laminin-coated six-well dishes. When applicable, total RNA was extracted using the RNeasy Mini Kit (Qiagen, Hilden, Germany) according to manufacturer instructions. Eighty nanograms of total RNA from each condition were used for each RT-qPCR reaction. PCRs were performed in the CFX96 Real-Time System (BioRad) using the iTaq Universal SYBER Green One-Step Kit (BioRad). PCRs were performed using human SMPD1-specific primers: forward (5'-GCTGGAATTATTACCGAAT-3') and reverse (5'-TCATCATAGAAGACCTCAA-3'). Specific primers of human glyceraldehyde-3-phosphate dehydrogenase (*GAPDH*): forward (5'-TGCACCACCAACTGCTTAG-3') and reverse (5'-GGATGCAGGGATGATGTTC-3') were used as internal control. RNA quantification was accomplished using the Bio-Rad CFX Manager real-time detection system Software (version 3.1, Bio-Rad). Each sample was measured in triplicate. Relative fold change gene expression level was calculated with the formula 2^-(ΔΔCq).

### Statistical analysis with mouse and human cells

All experiments were performed at least three independent times: three separate mouse offspring for primary MN cultures, three separate cultures for fibroblast cell lines experiments, or three separate iPSCs differentiation cycles for human MNs. Values were expressed as mean ± estimated standard error of the mean (SEM). Statistical analysis was done using the GraphPad Prism, version 9.4.1 (GraphPad Software Inc, San Diego, CA, USA). Normality of the data was verified with the Shapiro-Wilk test. When the data distribution was normal, differences between two groups were evaluated by Student t-test, and differences between more than two groups with one-way ANOVA, with Tukey's or Bonferroni's multiple comparisons post-tests. When the data was not normally distributed, the non-parametric Mann-Whitney test was used to compare differences between two groups, and the ANOVA Kruskal-Wallis with Dunn's multiple comparisons test to compare more than two groups. Values were considered significant when p<0.05. Statistical significance shown with " " indicates that a parametric test was applied and with "#" indicates that a non-parametric test was used.

### RESULTS

### In silico discovery of modifiers for SMN gene expression

Gene expression of human *SMN1* and *SMN2* genes is similarly regulated. Both genes encode the SMN protein, part of a protein complex with the same name, which in turn participates in several pathways related to RNA processing such as pre-mRNA splicing, histone mRNA and rRNA processing, mRNA transport and regulation, and protein translocation (Tisdale and Pellizzoni, 2017, p. 7).

To find negative modifier genes that could increase expression of SMN genes, we initially searched for regulatory genes with an opposite expression profile to *SMN1.* For this purpose, the computational tool ASACO was used, which is able to find genes with similar or opposite expression profiles from already published and normalized transcriptomics experiments (Fig. 1).

Since the genes of the SMN complex and those of the pathways in which they participate were expected to have a similar expression profile to *SMN1,* the correlation of the expression profiles of these genes with respect to *SMN1* was measured, and it was found that in all cases there was a positive correlation to this gene (Fig. 2AB). Thus, to find *SMN1* negative modifier genes, we searched for regulatory genes that not only had an expression profile opposite to *SMN1,* but also against at least 3 other genes of the SMN complex or the SMN-related RNA processing pathways (Fig. 2C). In orderto continue with the designed pipeline, these genes must also have orthologues in *Caenorhabditis elegans,* where they were to be tested experimentally.

In addition, since the ultimate goal was the repositioning of inhibitory drugs that would allow the upregulation of *SMN1* expression, only genes that had known inhibitory drugs were selected. Thus, we were finally left with 9 candidate genes, which presented an expression profile opposite to *SMN1* (Fig. 2D, Table 1).

**Table 1. Genes found with expression profiles opposite to SMN1. Genes of the SMN complex different to SMN1 and genes of related pathways in which the candidate gene also appeared with an opposite expression profile are shown. The last two columns show the corresponding orthologous genes in C. elegans. Note that in some cases, the human gene has several orthologous genes in C. elegans.**

| **Human** | | | ***Caenorhabditis elegans*** | |
|---|---|---|---|---|
| **Ensembl ID** | **Gene name** | **SMN complex and related pathways** | **Wormbase ID** | **Gene name** |
| ENSG00000186635 | *ARAP1* | *ELA VL4, GEM IN 2, GEMIN8, LSM1,SNRPG* | WBGene00017760 | *F23H11.4* |
| ENSG00000101439 | *CST3* | *GEMIN2,SNRPD1,SNRPG* | WBGene00000534 | *cpi-1* |
| ENSG00000101439 | *CST3* | *GEMIN2,SNRPD1,SNRPG* | WBGene00000535 | *cpi-2* |
| ENSG00000148180 | *GSN* | *ELAVL4,GEMIN2,GEMIN7* | WBGene00010593 | *gsnl-1* |
| ENSG00000108840 | *HDACS* | *LSM6,LSM8,SNRPD1* | WBGene00001837 | *hda-4* |
| ENSG00000175115 | *PACS1* | *GEMIN2,LSM6,SNRPG* | WBGene00044077 | *pacs-1* |
| ENSG00000166311 | *SMPD1* | *GEMIN2,IGF2BP1,SNRPD1,SNRPF,SNRPG* | WBGene00000211 | *asm-1* |
| ENSG00000166311 | *SMPD1* | *GEMIN2, IGF2BP1,SNRPD1,SNRPF,SNRPG* | WBGene00000212 | *asm-2* |
| ENSG00000166311 | *SMPD1* | *GEMIN2,IGF2BP1,SNRPD1,SNRPF,SNRPG* | WBGene00000213 | *asm-3* |
| ENSG00000175662 | *TOM1L2* | *GEMIN2,HNRNPR,LSM5,SNRPD1,SNRPG* | WBGene00015561 | *C07A12.7* |
| ENSG00000157514 | *TSC22D3* | *GEMIN4,GEMIN5,GEMIN6, IGF2BP1,SNRPB,SN RPD1,SNRPF,SNRPG* | WBGene00011824 | *tsct-1* |
| ENSG00000136878 | *USP20* | *LSM3,LSM5,SNRPF,SNRPG* | WBGene00010702 | *usp-33* |

These 9 candidate genes had an expression profile opposite to *SMN1* in 34 different transcriptomics experiments, which involved heterogeneous conditions related to cancer, RNA metabolism, neurogenesis or viral infections. In all cases we found that when *SMN1* appeared overexpressed, the candidate gene was underexpressed (Fig. 2C). This suggested that inhibition of the candidate gene could potentially increase *SMN1* expression levels.

### Generation and characterization of a C. elegans strain that allows measurement of SMN-1 levels

To test the candidate *SMN1* negative modifier genes, a transgenic *C*. *elegans* strain was initially constructed that allowed measurement of the amount of SMN-1 protein in the whole animal. We generated this strain using CRISPR technology by introducing the coding sequence for the fluorescence m-Cherry protein at the 3' end of the *SMN1* gene homolog of *C*. *elegans, smn-1.*

Using this strain, we can observe that SMN-1 is expressed through all developmental stages with higher expression in embryos. In the other developmental stages, the expression level is higher in gonads, head and a row compatible with being expressed in the central nerve cord (Fig. 3A-F). mCherry-SMN-1 protein appears mostly as discrete aggregates, which is consistent with previous results of overexpression in human cells corresponding to accumulations of the SMN complex (Sleeman et al., 2003).

We decided to evaluate the level of SMN-1 by measuring the fluorescence in the head of the nematode at the larval stage 4 (L4), the last larval stage before they develop into a fertile adult. To test if we can detect significant changes in the expression, we compare the fluorescence in normal condition with animals treated with RNAi of *smn-1.* As expected, the fluorescence levels was greatly reduced (Fig. 3 E-I).

### Inhibition of candidate genes: the acid sphingomyelinase 3 increases SMN-1 levels

In order to study the effect of suppression of the *in silica* candidate genes in the expression of SMN, we decided to knock-down those genes by RNAi feeding. For that, we used a commercially available library of double strand producing RNAi bacteria, where 11 of the 12 genes were present, missing the *asm-1* gene, one of the three homologs in *C*. *elegans* of human *SMPD1.* Among the 11 genes, inhibition of *asm-3* resulted in increased SMN-1 expression in all the four repetitions of the assay (Fig. 4).

### SMPD1 inhibitor drugs increase SMN-1 levels

The *asm-3* gene has at least three known inhibitory drugs: clomipramine, desipramine and amlodipine. So we wanted to know if the administration of these drugs can also increase the amount of SMN-1 protein. Interestingly, treatment with any of these three drugs generated an increase of the SMN-1 level, similarly to *asm-3* knockdown (Fig. 5). Moreover, this increase was usually proportional to the drug concentration used. Specifically, clomipramine significatively increased SMN-1 levels at a concentration of 60 µM and higher, desipramine from 250 µM, and amlodipine from 100 µM. At the concentrations tested, desipramine and amlodipine caused a delay in nematode development, which precluded testing at higher concentrations. However, treatment with clomipramine did not show a visible negative effect. Repetitions of the experiment with the 3 drugs on different days confirmed the result (Fig. 11).

In all previous experiments, nematodes were treated with the drug during all stages of development, from hatching to adulthood. To study the effect of clomipramine in the adult stage, without having received the drug before, clomipramine was administered for 48 hours in this stage of the nematode. The result was a significant increase in SMN-1 after this timely treatment (Fig. 6). Two repetitions of this experiment on different days showed a similar result (Fig. 12). The fluorescence measurement was repeated with a *C*. *elegans* strain that does not have SMN-1 fused to mCherry, to rule out effect on the mCherry fluorescence intensity upon the treatment. With this strain, no change in fluorescence was found when using the drug compared to untreated animals (Fig. 13).

### SMPD1 levels in human SMA fibroblasts are normal but treatment with clomipramine increases SMN levels

The *asm-3* gene is homologous to the human SMPD1 gene, and encodes a protein with sphingomyelin phosphodiesterase activity, located in the extracellular region and lysosomes (Staab et al., 2023). SMPD1 is associated with Niemann-Pick disease type A and B, a neurodegenerative disease characterized by the accumulation of sphingomyelin in lysosomes (Schuchman and Desnick, 2017). To explore whether the homologue of *asm-3* in humans, SMPD1, was altered in SMA cells, we cultured human fibroblast cell lines from an unaffected control (Control) and from SMA patients (SMAII and SMAI, see Materials and Methods). Total protein cell lysates of 2-day cultured fibroblasts were submitted to western blot analysis using anti-SMPD1 and anti-SMN antibodies. As expected, results showed that SMN protein level was significantly reduced in cell lysates from SMA fibroblasts (SMAII, 0.44±0.02; SMAI, 0.38±0.07; *p*<0.0001) compared to the control condition (Fig. 7A). Nonetheless, no significant differences of SMPD1 protein level between SMA (SMAII, 1.001±0.05; SMAI, 1.105±0.12; p>0.05) and control fibroblasts were observed. Besides, cultured fibroblasts were fixed and submitted to immunofluorescence protocol using the anti-SMPD1 antibody and relative fluorescence (CTCF, Corrected Total Cell Fluorescence) level of SMPD1 was measured. Results indicated no differences in SMPD1 level in SMA fibroblasts (CTCF: SMAII, 220.6±8.79 *p*=0.437; SMAI, 202.8±11.0; *p*=0.715) compared to control (CTCF: 207.0±11.39) (Fig. 7B). To analyze whether the level of *SMPD1* transcripts were altered in SMA fibroblasts, we quantified it by RT-qPCR *SMPD1* mRNA in 48 h cultured control and SMA cells. Total RNA was extracted and reverse-transcribed to cDNA, as a template to quantify *SMPD1* transcript level. *GAPDH* gene was used as a control. Again, *SMPD1* mRNA expression in SMA fibroblasts (SMAII, 1.35±0.252 *p*=0.307; SMAI, 0.87±0.21 *p*=0.884) was not significantly modified compared to the control (Fig. 7C). These results together indicated that SMPD1 protein and mRNA levels were not increased in SMA human fibroblasts compared to the control condition.

To elucidate the effect of clomipramine treatment on SMN and SMPD1 protein level in SMA fibroblasts, we examined by western blot (Fig. 7D) and immunofluorescence (Fig. 7E) experiments the outcome of adding this drug to the culture medium of SMAI and SMAII cultured cells. SMAII and SMAI fibroblasts were plated and cultured for 48 h. Medium was washed and a new fresh medium was added in the presence of an increased dose of clomipramine. Twenty-four hours later, protein extracts were obtained and submitted to western blot using anti-SMN antibody. Results indicated that SMN protein was significantly increased in 20µM clomipramine-treated SMAII (1.87±0.25; *p*=0.0112) and SMAI (1.76±0.149; *p*=0.0024) fibroblasts compared to control untreated condition (Fig. 7D). Addition of 5µM, 10µM, or 25µM clomipramine did not increase SMN protein level, but 20µM did. Thus, we examined SMN and SMPD1 levels in 20µM clomipramine-treated SMA fibroblasts by immunofluorescence. SMAII cells cultured in the presence of 20µM clomipramine or left untreated were fixed and submitted to immunofluorescence protocol using anti-SMN and anti-SMPD1 antibodies. Relative fluorescence levels of SMN and SMPD1 were measured in the cell soma. SMN was significantly increased in 20µM clomipramine treated SMAII fibroblasts (CTCF: 59.92±2.76; *p*<0.0001) compared to the untreated control (CTCF: 39.73±1.56) (upper panels and graph), whereas SMPD1 was significantly reduced in SMAII-treated (CTCF: 56.59±4.03; *p*<0.0001) fibroblasts compared to untreated condition (CTCF: 105.8±5.64) (bottom panels and graph) (Fig. 7E). Together these results indicated that clomipramine treatment reduced SMPD1 and increased SMN protein level in cultured SMA fibroblasts.

### SMPD1 levels are increased in mouse motor neurons and treatment with clomipramine increases the amount of SMN

To investigate SMPD1 protein level in SMA spinal cord MNs, cells were obtained from WT and SMNΔ7 genotyped 13-day mice embryos. After isolation, MNs were cultured for 6 days in the presence of a cocktail of neurotrophic factors. Total cell lysates were obtained and submitted to western blot analysis using anti-SMN and anti-SMPD1 antibodies. SMN level was significantly reduced in SMNΔ7 MNs (0.302±0.035; *p*<0.0001) compared to the WT control (Fig. 8A). When SMPD1 level was analyzed, results revealed a significant increase of SMPD1 protein in SMNΔ7 MNs (1.329±0.022; *p*=0.0286) compared to WT condition (Fig. 8A). Next, we performed immunofluorescence experiments to observe and measure SMPD1 protein in MN soma. Six-day cultured WT and SMNΔ7 MNs were fixed and processed for immunofluorescence analysis using anti-SMPD1 antibody. The CTCF was quantified and results showed increased SMPD1 level in cell soma of SMNΔ7 MNs (CTCF: 175.6±4.84; *p*<0.0001) compared to the WT control (CTCF: 123.6±4.02) (Fig. 8B). These results indicated that in cultured mouse SMNΔ7 MNs, the level of SMPD1 protein was increased.

To further explore alterations of SMPD1 protein in SMA MNs and the effect of clomipramine treatment on SMN levels, we decided to differentiate human iPSC cell lines to MNs. SMA and non-affected control (Control) human iPSCs (see Materials and Methods) were differentiated to MNs (de la Fuente et al., 2020; Du et al., 2015). Seven days after the differentiation period started in the presence of MN maturation medium, cells showed long neurites and MN markers were expressed, indicating that iPSCs were successfully differentiated to MNs (Fig. 14). Protein extracts of 7-day differentiated human SMAI (SMA type 1 patient), SMAII (SMA type 2 patient) and control MNs were submitted to western blot analysis using anti-SMN and anti-SMPD1 antibodies. SMN protein was significantly reduced in SMA MNs (SMAI 0.36±0.076; SMAII 0.33±0.033; *p*<0.0001) compared to the SMN level observed in the control (Fig. 9A). On the other hand, SMPD1 protein level was increased in SMA condition (SMAI 1.61±0.47; SMAII 1.50±0.13; *p*=0.029) compared to the control (Fig. 9A). Next, 7-day differentiated MNs were submitted to immunofluorescence protocol using anti-SMPD1 antibody, and CTCF was quantified. We observed an increase of SMPD1 fluorescence in SMA (CTCF: SMAI 104.0±4.38, *p*<0.0001; SMAII 84.96±2.99; *p*=0.0005) cells compared to control (CTCF: 70.87±1.78) (Fig. 9B). To explore whether SMPD1 protein rise in SMA MNs was associated with increased level of *SMPD1* transcript, *SMPD1* mRNA was quantified in 7-day differentiated control, SMAI and SMAII MNS by RT-qPCR. *GAPDH* gene was used as a control. *SMPD1* mRNA levels were increased in SMAI (3.22±0.54; *p*=0.0030) and SMAII (2.44±0.49; p=0.043) compared to control (Fig. 9D). Together these results indicated an increase of SMPD1 protein and *SMPD1* mRNA in human differentiated SMA MNs.

In order to analyze whether clomipramine treatment increased SMN protein in SMA human MNs, 7-day differentiated SMAI (Fig. 9D, left) and SMAII (Fig. 9D, right) were treated with three increased doses of clomipramine. Twenty-four hours later, total cell lysates were obtained and western blot was performed using anti-SMN antibody. SMN protein was significantly increased in 5µM clomipramine-treated SMAI MNs (1.23±0.09; *p*=0.021), and in 5µM or 10µM clomipramine-treated SMAII MNs (1.20±0.06; *p*=0.008; 1.34±0.1; *p*=0.0073, respectively) compared to the untreated control (Fig. 9D). No significant differences were observed in the remaining doses used. To examine the effect of clomipramine treatment on SMPD1 levels in SMA MNs, SMAI differentiated MNs were treated with 10µM clomipramine and fixed for immunofluorescence protocol using anti-SMPD1 and anti-SMN antibodies. CTCF of SMN and SMPD1 was measured in the cell soma. Clomipramine-treated SMA cells exhibited increased level of SMN (CTCF: 104.4±4.53; *p*<0.0001) and reduced level of SMPD1 (91.62±3.64, *p*<0.0001) compared to their respective untreated controls (SMN, 65.65±2.57; SMPD1, 126.7±5.74) (Fig. 9E). These results together suggest that clomipramine treatment increases SMN protein through SMPD1 reduction in differentiated human SMA MNs.

### REFERENCES

Achenbach, J., Saft, C., Faissner, S., 2021. Longitudinal Evaluation of the Effect of Tricyclic Antidepressants and Neuroleptics on the Course of Huntington's Disease-Data from a Real World Cohort. Brain Sci. 11, 413. https://doi.org/10.3390/brainsci11040413
Alayoubi, A.M., Wang, J.C.M., Au, B.C.Y., Carpentier, S., Garcia, V., Dworski, S., EI-Ghamrasni, S., Kirouac, K.N., Exertier, M.J., Xiong, Z.J., Privé, G.G., Simonaro, C.M., Casas, J., Fabrias, G., Schuchman, E.H., Turner, P.V., Hakem, R., Levade, T., Medin, J.A., 2013. Systemic ceramide accumulation leads to severe and varied pathological consequences. EMBO Mol. Med. 5, 827-842. https://doi.org/10.1002/emmm.201202301
Alessenko, A.V., Albi, E., 2020. Exploring Sphingolipid Implications in Neurodegeneration. Front. Neurol. 11.
Boda, B., Mas, C., Giudicelli, C., Nepote, V., Guimiot, F., Levacher, B., Zvara, A., Santha, M., LeGall, I., Simonneau, M., 2004. Survival motor neuron SMN1 and SMN2 gene promoters: identical sequences and differential expression in neurons and non-neuronal cells. Eur. J. Hum. Genet. EJHG 12, 729-737. https://doi.org/10.1038/sj.ejhg.5201217
Bolte, S., Cordelières, F.P., 2006. A guided tour into subcellular colocalization analysis in light microscopy. J. Microsc. 224, 213-232. https://doi.org/10.1111/j.1365-2818.2006.01706.x
Branchu, J., Biondi, O., Chali, F., Collin, T., Leroy, F., Mamchaoui, K., Makoukji, J., Pariset, C., Lopes, P., Massaad, C., Chanoine, C., Charbonnier, F., 2013. Shift from extracellular signal-regulated kinase to AKT/cAMP response element-binding protein pathway increases survival-motor-neuron expression in spinal-muscular-atrophy-like mice and patient cells. J. Neurosci. Off. J. Soc. Neurosci. 33, 4280-4294. https://doi.org/10.1523/JNEUROSCI.2728-12.2013
Briese, M., Esmaeili, B., Fraboulet, S., Burt, E.C., Christodoulou, S., Towers, P.R., Davies, K.E., Sattelle, D.B., 2009. Deletion of smn-1, the Caenorhabditis elegans ortholog of the spinal muscular atrophy gene, results in locomotor dysfunction and reduced lifespan. Hum. Mol. Genet. 18, 97-104. https://doi.org/10.1093/hmg/ddn320
Burghes, A.H.M., Beattie, C.E., 2009. Spinal muscular atrophy: why do low levels of survival motor neuron protein make motor neurons sick? Nat. Rev. Neurosci. 10, 597-609. https://doi.org/10.1038/nrn2670
Burghes, A.H.M., DiDonato, C.J., McGovern, V.L., Arnold, W.D., 2017. Chapter 15 - Mammalian Models of Spinal Muscular Atrophy, in: Sumner, C.J., Paushkin, S., Ko, C.-P. (Eds.), Spinal Muscular Atrophy. Academic Press, pp. 241-260. https://doi.org/10.1016/B978-0-12-803685-3.00015-X
Chen, E., Dixon, S., Naik, R., Noone, J.M., Buchenberger, J.D., Whitmire, S.M., Mills, R., Arnold, W., 2021. Early experiences of nusinersen for the treatment of spinal muscular atrophy: Results from a large survey of patients and caregivers. Muscle Nerve 63, 311-319. https://doi.org/10.1002/mus.27116
Chen, Q., Baird, S.D., Mahadevan, M., Besner-Johnston, A., Farahani, R., Xuan, J., Kang, X., Lefebvre, C., Ikeda, J.E., Korneluk, R.G., MacKenzie, A.E., 1998. Sequence of a 131-kb region of 5q13.1 containing the spinal muscular atrophy candidate genes SMN and NAIP. Genomics 48, 121-127. https://doi.org/10.1006/geno.1997.5141
Choi, B.J., Park, K.H., Park, M.H., Huang, E.J., Kim, S.H., Bae, J.-S., Jin, H.K., 2022. Acid sphingomyelinase inhibition improves motor behavioral deficits and neuronal loss in an amyotrophic lateral sclerosis mouse model. BMB Rep. 55, 621-626. https://doi.org/10.5483/BMBRep.2022.55.12.142
Chong, L.C., Gandhi, G., Lee, J.M., Yeo, W.W.Y., Choi, S.-B., 2021. Drug Discovery of Spinal Muscular Atrophy (SMA) from the Computational Perspective: A Comprehensive Review. Int. J. Mol. Sci. 22, 8962. https://doi.org/10.3390/ijms22168962
Connock, M., Andronis, L., Auguste, P., Dussart, C., Armoiry, X., 2020. Will the US$5 million onasemnogene abeparvosec treatment for spinal muscular atrophy represent "value for money" for the NHS? A rapid inquiry into suggestions that it may be cost-effective. Expert Opin. Biol. Ther. 20, 823-827. https://doi.org/10.1080/14712598.2020.1772747
Coratti, G., Cutrona, C., Pera, M.C., Bovis, F., Ponzano, M., Chieppa, F., Antonaci, L., Sansone, V., Finkel, R., Pane, M., Mercuri, E., 2021. Motor function in type 2 and 3 SMA patients treated with Nusinersen: a critical review and meta-analysis. Orphanet J. Rare Dis. 16, 430. https://doi.org/10.1186/s13023-021-02065-z
Dangouloff, T., Botty, C., Beaudart, C., Servais, L., Hiligsmann, M., 2021. Systematic literature review of the economic burden of spinal muscular atrophy and economic evaluations of treatments. Orphanet J. Rare Dis. 16, 47. https://doi.org/10.1186/s13023-021-01695-7
   de la Fuente, S., Sansa, A., Hidalgo, I., Vivancos, N., Romero-Guevara, R., Garcera, A., Soler, R.M., 2020. Calpain system is altered in survival motor neuron-reduced cells from in vitro and in vivo spinal muscular atrophy models. Cell Death Dis. 11, 487. https://doi.org/10.1038/s41419-020-2688-5
Deguise, M.-O., Baranello, G., Mastella, C., Beauvais, A., Michaud, J., Leone, A., De Amicis, R., Battezzati, A., Dunham, C., Selby, K., Warman Chardon, J., McMillan, H.J., Huang, Y.-T., Courtney, N.L., Mole, A.J., Kubinski, S., Claus, P., Murray, L.M., Bowerman, M., Gillingwater, T.H., Bertoli, S., Parson, S.H., Kothary, R., 2019a. Abnormal fatty acid metabolism is a core component of spinal muscular atrophy. Ann. Clin. Transl. Neurol. 6, 1519-1532. https://doi.org/10.1002/acn3.50855 Deguise, M.-O., Chehade, L., Tierney, A., Beauvais, A., Kothary, R., 2019b. Low fat diets increase survival of a mouse model of spinal muscular atrophy. Ann. Clin. Transl. Neurol. 6, 2340-2346. https://doi.org/10.1002/acn3.50920
Du, Z.-W., Chen, H., Liu, H., Lu, J., Qian, K., Huang, C.-L., Zhong, X., Fan, F., Zhang, S.-C., 2015. Generation and expansion of highly pure motor neuron progenitors from human pluripotent stem cells. Nat. Commun. 6, 6626. https://doi.org/10.1038/ncomms7626
Faissner, S., Mishra, M., Kaushik, D.K., Wang, J., Fan, Y., Silva, C., Rauw, G., Metz, L., Koch, M., Yong, V.W., 2017. Systematic screening of generic drugs for progressive multiple sclerosis identifies clomipramine as a promising therapeutic. Nat. Commun. 8, 1990. https://doi.org/10.1038/s41467-017-02119-6
Fallini, C., Bassell, G.J., Rossoll, W., 2012. Spinal muscular atrophy: the role of SMN in axonal mRNA regulation. Brain Res. 1462, 81-92. https://doi.org/10.1016/j.brainres.2012.01.044
Filosto, M., Aureli, M., Castellotti, B., Rinaldi, F., Schiumarini, D., Valsecchi, M., Lualdi, S., Mazzotti, R., Pensato, V., Rota, S., Gellera, C., Filocamo, M., Padovani, A., 2016. ASAH1 variant causing a mild SMA phenotype with no myoclonic epilepsy: a clinical, biochemical and molecular study. Eur. J. Hum. Genet. 24, 1578-1583. https://doi.org/10.1038/ejhg.2016.28
Fischer, U., Liu, Q., Dreyfuss, G., 1997. The SMN-SIP1 complex has an essential role in spliceosomal snRNP biogenesis. Cell 90, 1023-1029. https://doi.org/10.1016/s0092-8674(00)80368-2
Galper, J., Dean, N.J., Pickford, R., Lewis, S.J.G., Halliday, G.M., Kim, W.S., Dzamko, N., 2022. Lipid pathway dysfunction is prevalent in patients with Parkinson's disease. Brain J. Neurol. 145, 3472-3487. https://doi.org/10.1093/brain/awac176
Garcera, A., Mincheva, S., Gou-Fabregas, M., Caraballo-Miralles, V., Lladó, J., Comella, J.X., Soler, R.M., 2011. A new model to study spinal muscular atrophy: neurite degeneration and cell death is counteracted by BCL-X(L) Overexpression in motoneurons. Neurobiol. Dis. 42, 415-426. https://doi.org/10.1016/j.nbd.2011.02.003
Genabai, N.K., Ahmad, S., Zhang, Z., Jiang, X., Gabaldon, C.A., Gangwani, L., 2015. Genetic inhibition of JNK3 ameliorates spinal muscular atrophy. Hum. Mol. Genet. 24, 6986-7004. https://doi.org/10.1093/hmg/ddv401
Gou-Fabregas, M., Garcera, A., Mincheva, S., Perez-Garcia, M.J., Comella, J.X., Soler, R.M., 2009. Specific vulnerability of mouse spinal cord motoneurons to membrane depolarization. J. Neurochem. 110, 1842-1854. https://doi.org/10.1111/j.1471-4159.2009.06278.x
Gulbins, E., Palmada, M., Reichel, M., Lüth, A., Böhmer, C., Amato, D., Müller, C.P., Tischbirek, C.H., Groemer, T.W., Tabatabai, G., Becker, K.A., Tripal, P., Staedtler, S., Ackermann, T.F., van Brederode, J., Alzheimer, C., Weller, M., Lang, U.E., Kleuser, B., Grassmé, H., Kornhuber, J., 2013. Acid sphingomyelinase-ceramide system mediates effects of antidepressant drugs. Nat. Med. 19, 934-938. https://doi.org/10.1038/nm.3214
Han, K.-J., Foster, D., Harhaj, E.W., Dzieciatkowska, M., Hansen, K., Liu, C.-W., 2016. Monoubiquitination of survival motor neuron regulates its cellular localization and Cajal body integrity. Hum. Mol. Genet. 25, 1392-1405. https://doi.org/10.1093/hmg/ddw021
Hosseinibarkooie, S., Schneider, S., Wirth, B., 2017. Advances in understanding the role of disease-associated proteins in spinal muscular atrophy. Expert Rev. Proteomics 14, 581-592. https://doi.org/10.1080/14789450.2017.1345631
Hurwitz, R., Ferlinz, K., Sandhoff, K., 1994. The tricyclic antidepressant desipramine causes proteolytic degradation of lysosomal sphingomyelinase in human fibroblasts. Biol. Chem. Hoppe. Seyler 375, 447-450. https://doi.org/10.1515/bchm3.1994.375.7.447
Husedzinovic, A., Neumann, B., Reymann, J., Draeger-Meurer, S., Chari, A., Erfle, H., Fischer, U., Gruss, O.J., 2015. The catalytically inactive tyrosine phosphatase HD-PTP/PTPN23 is a novel regulator of SMN complex localization. Mol. Biol. Cell 26, 161-171. https://doi.org/10.1091/mbc.E14-06-1151
Jodelka, F.M., Ebert, A.D., Duelli, D.M., Hastings, M.L., 2010. A feedback loop regulates splicing of the spinal muscular atrophy-modifying gene, SMN2. Hum. Mol. Genet. 19, 4906-4917. https://doi.org/10.1093/hmg/ddq425
Jones, I., He, X., Katouzian, F., Darroch, P.I., Schuchman, E.H., 2008. Characterization of common SMPD1 mutations causing types A and B Niemann-Pick disease and generation of mutation-specific mouse models. Mol. Genet. Metab. 95, 152-162. https://doi.org/10.1016/j.ymgme.2008.08.004
Kamath, R.S., Ahringer, J., 2003. Genome-wide RNAi screening in Caenorhabditis elegans. Methods San Diego Calif 30, 313-321. https://doi.org/10.1016/s1046-2023(03)00050-1
Kasprzyk, A., 2011. BioMart: driving a paradigm change in biological data management. Database J. Biol. Databases Curation 2011, bar049. https://doi.org/10.1093/database/bar049
Khalil, B., Morderer, D., Price, P.L., Liu, F., Rossoll, W., 2018. mRNP assembly, axonal transport, and local translation in neurodegenerative diseases. Brain Res. 1693, 75-91. https://doi.org/10.1016/j.brainres.2018.02.018
Kim, J.K., Gabel, H.W., Kamath, R.S., Tewari, M., Pasquinelli, A., Rual, J.-F., Kennedy, S., Dybbs, M., Bertin, N., Kaplan, J.M., Vidal, M., Ruvkun, G., 2005. Functional genomic analysis of RNA interference in C. elegans. Science 308, 1164-1167. https://doi.org/10.1126/science.1109267
Kim, W., Underwood, R.S., Greenwald, I., Shaye, D.D., 2018. OrthoList 2: A New Comparative Genomic Analysis of Human and Caenorhabditis elegans Genes. Genetics 210, 445-461. https://doi.org/10.1534/genetics.118.301307
Konieczny, P., Artero, R., 2020. Drosophila SMN2 minigene reporter model identifies moxifloxacin as a candidate therapy for SMA. FASEB J. Off. Publ. Fed. Am. Soc. Exp. Biol. 34, 3021-3036. https://doi.org/10.1096/fj.201802554RRR
Lanfranco, M., Cacciottolo, R., Borg, R.M., Vassallo, N., Juge, F., Bordonné, R., Cauchi, R.J., 2017. Novel interactors of the Drosophila Survival Motor Neuron (SMN) Complex suggest its full conservation. FEBS Lett. 591, 3600-3614. https://doi.org/10.1002/1873-3468.12853
Lee, J.K., Jin, H.K., Park, M.H., Kim, B., Lee, P.H., Nakauchi, H., Carter, J.E., He, X., Schuchman, E.H., Bae, J., 2014. Acid sphingomyelinase modulates the autophagic process by controlling lysosomal biogenesis in Alzheimer's disease. J. Exp. Med. 211, 1551-1570. https://doi.org/10.1084/jem.20132451
Lefebvre, S., Bürglen, L., Reboullet, S., Clermont, O., Burlet, P., Viollet, L., Benichou, B., Cruaud, C., Millasseau, P., Zeviani, M., 1995. Identification and characterization of a spinal muscular atrophy-determining gene. Cell 80, 155-165.
Lunn, M.R., Wang, C.H., 2008. Spinal muscular atrophy. Lancet 371, 2120-2133. https://doi.org/10.1016/50140-6736(08)60921-6
McCabe, E.R.B., 2017. Modifier genes: Moving from pathogenesis to therapy. Mol. Genet. Metab. 122, 1-3. https://doi.org/10.1016/j.ymgme.2017.05.018
McCluskey, G., Donaghy, C., Morrison, K.E., McConville, J., Duddy, W., Duguez, S., 2022. The Role of Sphingomyelin and Ceramide in Motor Neuron Diseases. J. Pers. Med. 12, 1418. https://doi.org/10.3390/jpm12091418
Mendell, J.R., Al-Zaidy, S., Shell, R., Arnold, W.D., Rodino-Klapac, L.R., Prior, T.W., Lowes, L., Alfano, L., Berry, K., Church, K., Kissel, J.T., Nagendran, S., L'ltalien, J., Sproule, D.M., Wells, C., Cardenas, J.A., Heitzer, M.D., Kaspar, A., Corcoran, S., Braun, L., Likhite, S., Miranda, C., Meyer, K., Foust, K.D., Burghes, A.H.M., Kaspar, B.K., 2017. Single-Dose Gene-Replacement Therapy for Spinal Muscular Atrophy. N. Engl. J. Med. 377, 1713-1722. https://doi.org/10.1056/NEJMoa1706198
Messina, S., 2018. New Directions for SMA Therapy. J. Clin. Med. 7. https://doi.org/10.3390/jcm7090251
Miguel-Aliaga, I., Culetto, E., Walker, D.S., Baylis, H.A., Sattelle, D.B., Davies, K.E., 1999. The Caenorhabditis elegans orthologue of the human gene responsible for spinal muscular atrophy is a maternal product critical for germline maturation and embryonic viability. Hum. Mol. Genet. 8, 2133-2143.
Mohassel, P., Donkervoort, S., Lone, M.A., Nalls, M., Gable, K., Gupta, S.D., Foley, A.R., Hu, Y., Saute, J.A.M., Moreira, A.L., Kok, F., Introna, A., Logroscino, G., Grunseich, C., Nickolls, A.R., Pourshafie, N., Neuhaus, S.B., Saade, D., Gangfuβ, A., Kölbel, H., Piccus, Z., Le Pichon, C.E., Fiorillo, C., Ly, C.V., Töpf, A., Brady, L., Specht, S., Zidell, A., Pedro, H., Mittelmann, E., Thomas, F.P., Chao, K.R., Konersman, C.G., Cho, M.T., Brandt, T., Straub, V., Connolly, A.M., Schara, U., Roos, A., Tarnopolsky, M., Höke, A., Brown, R.H., Lee, C.-H., Hornemann, T., Dunn, T.M., Bönnemann, C.G., 2021. Childhood amyotrophic lateral sclerosis caused by excess sphingolipid synthesis. Nat. Med. 27, 1197-1204. https://doi.org/10.1038/s41591-021-01346-1
Nagree, M.S., Rybova, J., Kleynerman, A., Ahrenhoerster, C.J., Saville, J.T., Xu, T., Bachochin, M., McKillop, W.M., Lawlor, M.W., Pshezhetsky, A.V., Isaeva, O., Budde, M.D., Fuller, M., Medin, J.A., 2023. Spinal muscular atrophy-like phenotype in a mouse model of acid ceramidase deficiency. Commun. Biol. 6, 1-20. https://doi.org/10.1038/s42003-023-04932-w
Navascues, J., Bengoechea, R., Tapia, O., Casafont, I., Berciano, M.T., Lafarga, M., 2008. SUMO-1 transiently localizes to Cajal bodies in mammalian neurons. J. Struct. Biol. 163, 137-146. https://doi.org/10.1016/j.jsb.2008.04.013
O'Hern, P., Garcia, E.L., Hao, L.T., Hart, A.C., Matera, A.G., Beattie, C.E., 2017. Chapter 14 - Nonmammalian Animal Models of Spinal Muscular Atrophy, in: Sumner, C.J., Paushkin, S., Ko, C.-P. (Eds.), Spinal Muscular Atrophy. Academic Press, pp. 221-239. https://doi.org/10.1016/B978-0-12-803685-3.00014-8
Owen, N., Doe, C.L., Mellor, J., Davies, K.E., 2000. Characterization of the Schizosaccharomyces pombe orthologue of the human survival motor neuron (SMN) protein. Hum Mol Genet 9, 675-684. https://doi.org/10.1093/hmg/9.5.675
Pane, M., Coratti, G., Sansone, V.A., Messina, S., Catteruccia, M., Bruno, C., Sframeli, M., Albamonte, E., Pedemonte, M., Brolatti, N., Mizzoni, I., D'Amico, A., Bravetti, C., Berti, B., Palermo, C., Leone, D., Salmin, F., De Sanctis, R., Pera, M.C., Piastra, M., Genovese, O., Ricci, F., Cavallina, I., Masson, R., Zanin, R., Agosto, C., Salomon, E., Bruno, I., Magnolato, A., Bertini, E., Tiziano, F.D., Bovis, F., Mercuri, E., Italian EAP Working Group, 2023. Type I spinal muscular atrophy patients treated with nusinersen: 4-year follow-up of motor, respiratory and bulbar function. Eur. J. Neurol. 30, 1755-1763. https://doi.org/10.1111/ene.15768
Pérez-Pulido, A.J., Asencio-Cortés, G., Brokate-Llanos, A.M., Brea-Calvo, G., Rodríguez-Grinolo, R., Garzón, A., Muñoz, M.J., 2021. Serial co-expression analysis of host factors from SARS-CoV viruses highly converges with former high-throughput screenings and proposes key regulators. Brief. Bioinform. 22, 1038-1052. https://doi.org/10.1093/bib/bbaa419
Petit, C.S., Lee, J.J., Boland, S., Swarup, S., Christiano, R., Lai, Z.W., Mejhert, N., Elliott, S.D., McFall, D., Haque, S., Huang, E.J., Bronson, R.T., Harper, J.W., Farese, R.V., Walther, T.C., 2020. Inhibition of sphingolipid synthesis improves outcomes and survival in GARP mutant wobbler mice, a model of motor neuron degeneration. Proc. Natl. Acad. Sci. 117, 10565-10574. https://doi.org/10.1073/pnas.1913956117
Pieragostino, D., Cicalini, I., Lanuti, P., Ercolino, E., di loia, M., Zucchelli, M., Zappacosta, R., Miscia, S., Marchisio, M., Sacchetta, P., Onofrj, M., Del Boccio, P., 2018. Enhanced release of acid sphingomyelinase-enriched exosomes generates a lipidomics signature in CSF of Multiple Sclerosis patients. Sci. Rep. 8, 3071. https://doi.org/10.1038/s41598-018-21497-5
Ratni, H., Ebeling, M., Baird, J., Bendels, S., Bylund, J., Chen, K.S., Denk, N., Feng, Z., Green, L., Guerard, M., Jablonski, P., Jacobsen, B., Khwaja, O., Kletzl, H., Ko, C.-P., Kustermann, S., Marquet, A., Metzger, F., Mueller, B., Naryshkin, N.A., Paushkin, S.V., Pinard, E., Poirier, A., Reutlinger, M., Weetall, M., Zeller, A., Zhao, X., Mueller, L., 2018. Discovery of Risdiplam, a Selective Survival of Motor Neuron-2 ( SMN2) Gene Splicing Modifier for the Treatment of Spinal Muscular Atrophy (SMA). J. Med. Chem. 61, 6501-6517. https://doi.org/10.1021/acs.jmedchem.8b00741
Rhein, C., Zoicas, I., Marx, L.M., Zeitler, S., Hepp, T., von Zimmermann, C., Mühle, C., Richter-Schmidinger, T., Lenz, B., Erim, Y., Reichel, M., Gulbins, E., Kornhuber, J., 2021. mRNA Expression of SMPD1 Encoding Acid Sphingomyelinase Decreases upon Antidepressant Treatment. Int. J. Mol. Sci. 22, 5700. https://doi.org/10.3390/ijms22115700
Rizzo, F., Nizzardo, M., Vashisht, S., Molteni, E., Melzi, V., Taiana, M., Salani, S., Santonicola, P., Di Schiavi, E., Bucchia, M., Bordoni, A., Faravelli, I., Bresolin, N., Comi, G.P., Pozzoli, U., Corti, S., 2019. Key role of SMN/SYNCRIP and RNA-Motif 7 in spinal muscular atrophy: RNA-Seq and motif analysis of human motor neurons. Brain 142, 276-294. https://doi.org/10.1093/brain/awy330
Rochette, C.F., Gilbert, N., Simard, L.R., 2001. SMN gene duplication and the emergence of the SMN2 gene occurred in distinct hominids: SMN2 is unique to Homo sapiens. Hum. Genet. 108, 255-266.
Sansa, A., de la Fuente, S., Comella, J.X., Garcera, A., Soler, R.M., 2021. Intracellular pathways involved in cell survival are deregulated in mouse and human spinal muscular atrophy motoneurons. Neurobiol. Dis. 155, 105366. https://doi.org/10.1016/j.nbd.2021.105366
Schellino, R., Boido, M., Borsello, T., Vercelli, A., 2018. Pharmacological c-Jun NH2-Terminal Kinase (JNK) Pathway Inhibition Reduces Severity of Spinal Muscular Atrophy Disease in Mice. Front. Mol. Neurosci. 11, 308. https://doi.org/10.3389/fnmol.2018.00308
Schilling, M., Prusty, A.B., Boysen, B., Oppermann, F.S., Riedel, Y.L., Husedzinovic, A., Rasouli, H., König, A., Ramanathan, P., Reymann, J., Erfle, H., Daub, H., Fischer, U., Gruss, O.J., 2021. TOR signaling regulates liquid phase separation of the SMN complex governing snRNP biogenesis. Cell Rep. 35, 109277. https://doi.org/10.1016/j.celrep.2021.109277
Schindelin, J., Arganda-Carreras, I., Frise, E., Kaynig, V., Longair, M., Pietzsch, T., Preibisch, S., Rueden, C., Saalfeld, S., Schmid, B., Tinevez, J.-Y., White, D.J., Hartenstein, V., Eliceiri, K., Tomancak, P., Cardona, A., 2012. Fiji: an open-source platform for biological-image analysis. Nat. Methods 9, 676-682. https://doi.org/10.1038/nmeth.2019
Schmid, A., DiDonato, C.J., 2007. Animal models of spinal muscular atrophy. J. Child Neurol. 22, 1004-1012. https://doi.org/10.1177/0883073807305667
Schmitt, F., Hussain, G., Dupuis, L., Loeffler, J.-P., Henriques, A., 2014. A plural role for lipids in motor neuron diseases: energy, signaling and structure. Front. Cell. Neurosci. 8, 25. https://doi.org/10.3389/fnce1.2014.00025
Schuchman, E.H., Desnick, R.J., 2017. Types A and B Niemann-Pick disease. Mol. Genet. Metab. 120, 27-33. https://doi.org/10.1016/j.ymgme.2016.12.008
Schuchman, E.H., Suchi, M., Takahashi, T., Sandhoff, K., Desnick, R.J., 1991. Human acid sphingomyelinase. Isolation, nucleotide sequence and expression of the full-length and alternatively spliced cDNAs. J. Biol. Chem. 266, 8531-8539.
Sleeman, J.E., Trinkle-Mulcahy, L., Prescott, A.R., Ogg, S.C., Lamond, A.I., 2003. Cajal body proteins SMN and Coilin show differential dynamic behaviour in vivo. J. Cell Sci. 116, 2039-2050. https://doi.org/10.1242/jcs.00400
Sleigh, J.N., Buckingham, S.D., Esmaeili, B., Viswanathan, M., Cuppen, E., Westlund, B.M., Sattelle, D.B., 2010. A novel Caenorhabditis elegans allele smn-1(cb131), mimicking a mild form of spinal muscular atrophy, provides a convenient drug screening platform highlighting new and pre-approved compounds. Hum. Mol. Genet. https://doi.org/10.1093/hmg/ddq459
Staab, T.A., McIntyre, G., Wang, L., Radeny, J., Bettcher, L., Guillen, M., Peck, M.P., Kalil, A.P., Bromley, S.P., Raftery, D., Chan, J.P., 2023. The lipidomes of C. elegans with mutations in asm-3/acid sphingomyelinase and hyl-2/ceramide synthase show distinct lipid profiles during aging. Aging 15, 650-674. https://doi.org/10.18632/aging.204515
Stiernagle, T., 2006. Maintenance of C. elegans. WormBook Online Rev. C Elegans Biol. 1-11. https://doi.org/10.1895/wormbook.1.101.1
Talbot, K., Tizzano, E.F., 2017. The clinical landscape for SMA in a new therapeutic era. Gene Ther. 24, 529-533. https://doi.org/10.1038/gt.2017.52
Tisdale, S., Pellizzoni, L., 2017. Chapter 7 - RNA-Processing Dysfunction in Spinal Muscular Atrophy, in: Sumner, C.J., Paushkin, S., Ko, C.-P. (Eds.), Spinal Muscular Atrophy. Academic Press, pp. 113-131. https://doi.org/10.1016/B978-0-12-803685-3.00007-0
Toman, R.E., Movsesyan, V., Murthy, S.K., Milstien, S., Spiegel, S., Faden, A.I., 2002. Ceramide-induced cell death in primary neuronal cultures: upregulation of ceramide levels during neuronal apoptosis. J. Neurosci. Res. 68, 323-330. https://doi.org/10.1002/jnr.10190
Wirth, B., Brichta, L., Hahnen, E., 2006. Spinal muscular atrophy: from gene to therapy. Semin. Pediatr. Neurol. 13, 121-131. https://doi.org/10.1016/j.spen.2006.06.008
Wishart, D.S., Feunang, Y.D., Guo, A.C., Lo, E.J., Marcu, A., Grant, J.R., Sajed, T., Johnson, D., Li, C., Sayeeda, Z., Assempour, N., Iynkkaran, I., Liu, Y., Maciejewski, A., Gale, N., Wilson, A., Chin, L., Cummings, R., Le, D., Pon, A., Knox, C., Wilson, M., 2018. DrugBank 5.0: a major update to the DrugBank database for 2018. Nucleic Acids Res. 46, D1074-D1082. https://doi.org/10.1093/nar/gkx1037

## Claims

1. A composition comprising an inhibitory molecule capable of inhibiting the activity or expression of the gene SMPD1 (Sphingomyelin phosphodiesterase 1), for use in the treatment of Spinal Muscular Atrophy (SMA) in a subject in need thereof.

2. The composition for use according to claim 1, wherein the molecule is an acid sphingomyelinase (ASM) expression inhibitor.

3. The composition for use according to claim 2, wherein the ASM inhibitor may be at least one selected from the group consisting of a tricyclic antidepressant, a peptide, a peptide mimetic, a substrate analogue, an aptamer, and an antibody or antibody fragment, specifically binding to ASM protein.

4. The composition for use according to claim 3, wherein the ASM inhibitor is selected from the group consisting of clomipramine, desipramine, amitriptyline, amlodipine, astemizole, benzatropine, citalopram, clomiphene, cloperastine, cyclobenzaprine, cyproheptadine, diphenhydramine, fexofenadine, fluoxetine, maprotiline, nortriptyline, paroxetine, promethazine, sertraline, and zoledronic acid.

5. The composition for use according to claim 3, wherein the molecule is selected from the list consisting of clomipramine, Desipramine and Amlodipine.

6. The composition for use according to claim 3, wherein the ASM expression inhibitor is clomipramine.

7. The composition for use according to claim 2, wherein the ASM expression inhibitor is selected from a group consisting of an antisense nucleotide, small hairpin RNA (shRNA), small interfering (siRNA), microRNA (miRNA) and ribozyme, complementarily binding to mRNA of an ASM gene or a gene promoting expression of ASM.

8. The composition for use according to any one of claims 1 to 7, wherein the composition is a pharmaceutical composition optionally further comprising a carrier and/or one or more pharmaceutically acceptable excipients.

9. The composition for use according to any one of the precedent claims, wherein the treatment is a palliative treatment of one or more symptoms of Spinal Muscular Atrophy (SMA).

10. The composition for use according to any one of the precedent claims, wherein the treatment is a palliative treatment of one or more muscular disorders that are part of the one or more symptoms of Spinal Muscular Atrophy (SMA).
